(19) Europäisches Patentamt
European Patent Office
Office européen des brevets

(11) **EP 4 706 676 A1**

(12) **EUROPEAN PATENT APPLICATION**

(43) Date of publication:
**11.03.2026 Bulletin 2026/11**

(21) Application number: **24199456.5**

(22) Date of filing: **10.09.2024**

(51) International Patent Classification (IPC):
**A61K 40/11** (2025.01) **A61K 40/31** (2025.01)
**A61K 40/42** (2025.01) **C07K 16/28** (2006.01)
**C07K 16/30** (2006.01)

(52) Cooperative Patent Classification (CPC):
**A61K 40/4211; A61K 40/42; A61K 40/4258;**
A61K 2239/13; A61K 2239/17; A61K 2239/47;
A61K 2239/48; A61K 2239/49; C07K 16/2803;
C07K 16/3084; C07K 2317/622

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Max-Planck-Gesellschaft zur
Förderung der
Wissenschaften e.V.
80539 München (DE)**

(72) Inventor: **The designation of the inventor has not
yet been filed**

(74) Representative: **Wichmann, Hendrik
Wuesthoff & Wuesthoff
Patentanwälte und Rechtsanwalt PartG mbB
Schweigerstraße 2
81541 München (DE)**

Remarks:
The complete document including Reference
Table(s) and the Sequence Listing(s) can be
downloaded from the EPO website

(54) **CYSTEINE-ENGINEERED ANTIGEN-BINDING DOMAINS**

(57) The invention pertains to an antigen-binding domain, which comprise a heavy chain variable region and optionally a light chain variable region. The antigen-binding domain is capable of binding to an antigen. At least one of the complementarity-determining regions contains an amino acid residue, other than cysteine, with a solvent-accessible surface area ranging from 0.00 to 0.75 and is located at a distance of 3 to 14 A from the antigen and is substituted to a thiol containing amino acid. This unique configuration enhances the antigen-binding domain antigen-binding and cytotoxicity capabilities.

EP 4 706 676 A1

## Description

### Background of invention

Immunotherapy represents a key frontier in the development of novel therapeutics. Antibodies and antibody fragments play a central role as they allow specific targeting of cells. Antigen-binding domains can be part of therapeutic antibodies such as monoclonal antibodies (mAbs), chimeric antibodies, humanized antibodies, fully human antibodies, or bispecific antibodies, as well as antibody fragments like Fabs (fragment antigen-binding), scFv (Single-chain variable fragment), nanobodies (VHH domains), or antibody-drug conjugates (ADCs).[14] Furthermore, antigen-binding domains may also be part of engineered antibodies with enhanced effector functions, such as improved antibody-dependent cell-mediated cytotoxicity (ADCC) or complement-dependent cytotoxicity (CDC), or as T cell-engaging bispecific antibodies that simultaneously bind to antigens on tumor cells and distinct subunits on T cells.[15] In particular, therapeutic antibodies revolutionized cancer treatment in the last decades, with notable examples including rituximab for B-cell lymphomas, daratumumab for multiple myeloma, Trastuzumab (Herceptin) and Pertuzumab (Perjeta) for breast cancer, and Bevacizumab (Avastin) for colorectal cancer.[16, 17]

Despite the remarkable achievements of the use of therapeutic antibodies, the technology still faces several challenges. These include adverse effects like neurotoxicity or cytokine release syndrome. Moreover, as mostly targeting a single type of tumor-associated antigen, antibody therapy leads to emerging resistance of the cancer cells through antigen loss or downregulation, mutations in the mAb binding site, or even shedding of the target to the patient serum that blocks further administrated antibodies from reaching their target cells.[1-3 18, 19] These limitations highlighted the need to target multiple antigens on cancer cells, which initiated the development of multi-specific antibody constructs. One promising alternative strategy for cancer immunotherapy is to co-target the cellular microenvironment. This approach not only expands the target scope but also minimizes the risk of evasion mechanisms.[4, 5] A common feature of several cancer types, including BCL, is a reduced cellular microenvironment, caused by differential expression of disulfide-active enzymes, such as protein disulfide isomerase (PDI) or the thioredoxin system.[6-10] These alterations can contribute substantially to disease progression. For example, reduced thiol groups on breast cancer cells facilitate attachment to the endothelium, thereby promoting metastasis.[9] Furthermore, overexpression of PDI is associated with cancer growth and drug resistance.[11,12] In addition to these effects to cancer biology, the reduced cellular microenvironment is also an attractive target for therapy. Small molecule PDI inhibitors have been reported to reduce metastasis in breast cancer and prolong the survival of mice transplanted with multiple myeloma.[9,13]

The authors recently demonstrated that abnormal cell membrane redox states on several blood and breast cancer cell lines, including the MDA-MB-231 TNBC cell line, allow the targeting of multiple cell surface receptors simultaneously.[8] Specifically, a single-domain antibody (nanobody) derived from an alpaca heavy chain-only IgG was developed and shown to bind several subtypes of BCL and breast cancer.[8] This nanobody, denoted CB2, interacts solely with altered redox states of the cellular microenvironment via the non-canonical cysteine 105 in the complementarity-determining region (CDR) 3. However, while thiol-mediated uptake of CB2 enables its use as an intracellular drug carrier, a fraction of the nanobody remains bound to the cell surface, indicating binding to multiple types of cell surface receptors.[8]

### Summary of the invention

The invention is defined in the claims. The invention provides an antigen-binding domain that binds specifically to target cells in an antigen-dependent manner and/or additionally in a thiol-dependent manner, enabling effective treatment of cancers, also those undergoing antigen escape. The antigen-binding domain comprises at least one amino acid residue other than cysteine, which is substituted to a thiol group containing amino acid. This invention offers a solution to the limitations of current immunotherapies, such as antigen escape, providing a more effective approach to disease treatment, including cancer treatment that is feasible in any antigen-binding configuration, derived from native panning of antibody or antibody-fragment or synthetically designed in silico.

In particular, the development of a novel immunotherapy with cysteine-engineered antigen-binding fragments is disclosed, thus directly targeting the cellular microenvironment instead of a single antigen. For example, the inventors show that CB2-antigen-binding domain (as part of a CAR) can trigger T cell activation and cytotoxicity against different subtypes of B-cell lymphoma (BCL), while healthy human Peripheral Blood Mononuclear Cells (PBMCs) are not targeted. Furthermore, the inventors show CB2-CAR-T cell activity against antigen escape models that are resistant to conventional CAR-T therapies. In addition, the inventors validate the results by cysteine engineering additional non-cancer-related nanobody-CAR, effectively initiating cancer targeting. Finally, the inventors demonstrate the method's ability to target and generate destruction of cancer cells through cysteine engineering by an antigen-dependent and a thiol-dependent binding by a single CAR construct comprising the antigen-binding domain of the clinically used CAR-T-cell therapy Kymriah® (anti-CD19 CAR). Thus, by introducing one or more thiol groups containing amino acids, preferably one, at positions on the anti-CD19 scFv FMC63, the inventors evaluated the CAR-T cell antigen-dependent and independent cytotoxicity and showed how cysteine-engineered antigen-binding domain as part of the FMC63-CAR-T cells demonstrate high efficacy against CD19-positive and negative lymphoma, both *in vitro* and in xenograft mouse models.

Hence, the present invention provides an antigen-binding domain that not only binds specifically to the target but also in a thiol-dependent way. This is particularly advantageous as cancer cells often have an altered redox potential due to altered expression of enzymes regulating cell membrane redox system. The thiol-dependent targeting of the antigen-binding domain makes it particularly suited for cancers undergoing antigen escape. The antigen-binding domain according to the present invention efficiently target diseased cells such as cancer cells. This targeting has been experimentally shown e.g. for an anti-CD19 antigen-binding domain as part of a CAR. Additionally, the inventors provide a clear rationale for the criteria to be met for thiol group containing amino acid substitution based on solvent-accessible surface area and distance to the target. Importantly, when the cysteine is substituted with a serine, the cytotoxic effect in the antigen-binding domain as part of a CAR T-cell is lost, highlighting the critical role of the thiol group containing amino acid in this invention. Example 3 herein provides experimental evidence that the replacement of the cysteine by serine can lead to a complete loss of the T cells cytotoxic activity.

The invention provides an antigen-binding domain that comprises at least one amino acid residue other than cysteine, which has a solvent-accessible surface area from 0.00 to 0.75 and is located at a distance to the antigen from 3 to 14 Å. This amino acid residue is substituted to a thiol group containing amino acid, enabling the antigen-binding domain to bind to the target in a thiol-dependent manner.

The invention offers several advantages over the current state of the art. For example, the thiol-dependent targeting of the antigen-binding domain allows for the effective treatment of cancers undergoing antigen escape, a significant challenge in current cancer treatments.

Furthermore, the antigen-binding domain may be part of an antibody, wherein the antibody comprises an Fc domain.

**Description of the Figures**

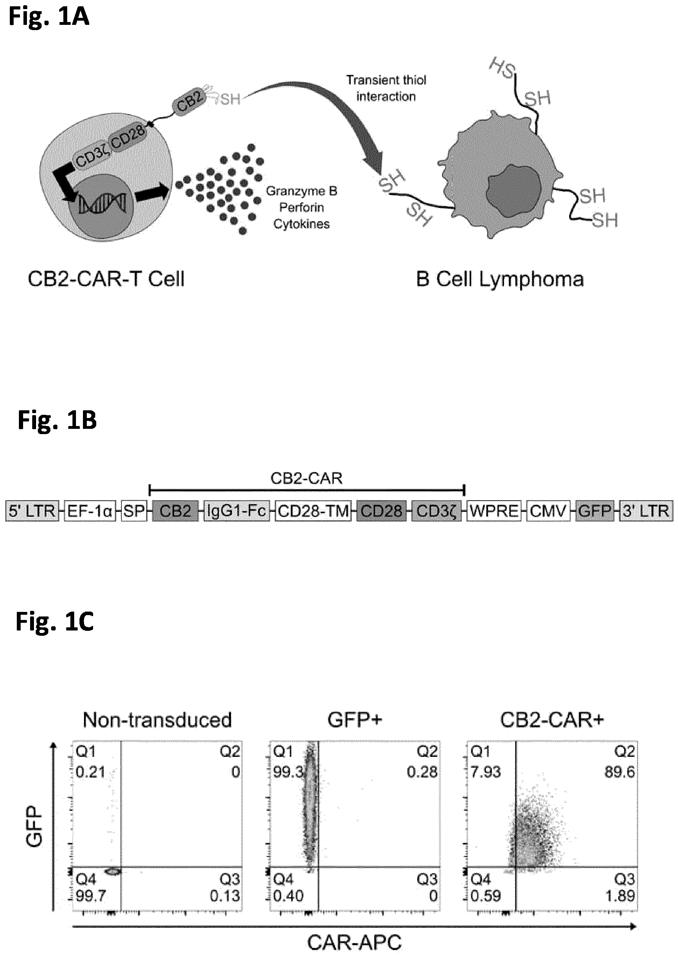

**Fig. 1A** Generation of CB2-CAR-T cells. Schematic representation of thiol-dependent CB2-CAR-T cell activity against BCL.

**Fig. 1B** Domain structure of CB2-CD28-CAR_GFP construct. Abbreviations: LTR long terminal repeat, EF-1$\alpha$ - human elongation factor-1 alpha promotor, SP - signal peptide, IgG1-Fc - human IgG1-Fc hinge, CD28-TM - CD28 transmembrane domain, CD28 - CD28 costimulatory domain, CD3$\zeta$ - CD3$\zeta$ signaling domain, WPRE - Woodchuck Hepatitis Virus Posttranscriptional Regulatory Element, CMV - human cytomegalovirus enhancer and promotor, GFP - green fluorescent protein.

**Fig. 1C** Determination of transduction efficiencies of GFP control and CB2-CAR-T cells on day 6 after transduction. Representative plots are shown. Cells were stained with MonoRab™ Rabbit Anti-Camelid VHH Cocktail iFluor 647 and measured on a FACSCanto™ II Flow Cytometry System detecting antibody fluorescence (CAR-APC) and GFP fluorescence (GFP). Percentages of the individual populations are indicated in the graphs. (

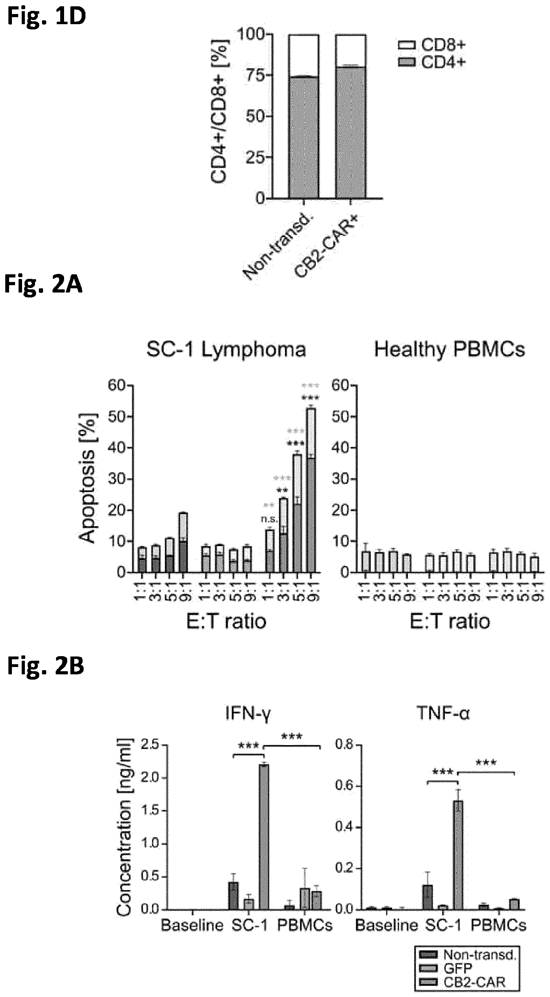

**Fig. 1D** Immunophenotyping of non-transduced and CB2-CART cells. Cells were stained with PE anti-human CD4 Antibody and FITC anti-human CD8 Antibody and measured on a FACSCanto™ II Flow Cytometry System. Grey bars represent percentage of CD4+ and white bars of CD8+ T cells. Mean values $\pm$ SEM of n=3 replicates are shown.

**Fig. 2A** CB2-CAR-T cells mediate cytotoxicity against B cell lymphoma and undergo T cell activation. Flow cytometry-based apoptosis assays. SC-1 lymphoma or healthy human PBMCs were incubated for 72 hours with CB2-CAR or control T cells at different E:T ratios. Apoptosis was assessed by incubating with Annexin V-PE (early apoptosis) and 7-AAD (late apoptosis) and measuring on a FACSCanto™ II Flow Cytometry System. Stacked bars represent the different types of apoptosis (darker bottom part of bars for late apoptosis and lighter top part for early apoptosis). Mean values $\pm$ SEM of n=3 replicates are shown. Significance was assessed via 2-way ANOVA followed by Tukey's post-hoc test. Bottom significance levels refer to differences in late apoptosis and top significance levels to differences in early apoptosis compared to both controls (in the case of different significance levels, the less significant is indicated).

**Fig. 2B** Cytokine secretion assays. Levels of IFN-$\gamma$ and TNF-$\alpha$ in co-culture supernatants after 72 hours were determined via ELISA. T cells were co-incubated with SC-1 cells or PBMCs respectively. Baseline samples represent T cells cultured without target cells under the same conditions. Mean values $\pm$ SEM of n=3 replicates are shown, and significance of differences was assessed via 2-way ANOVA followed by Tukey's post-hoc test.

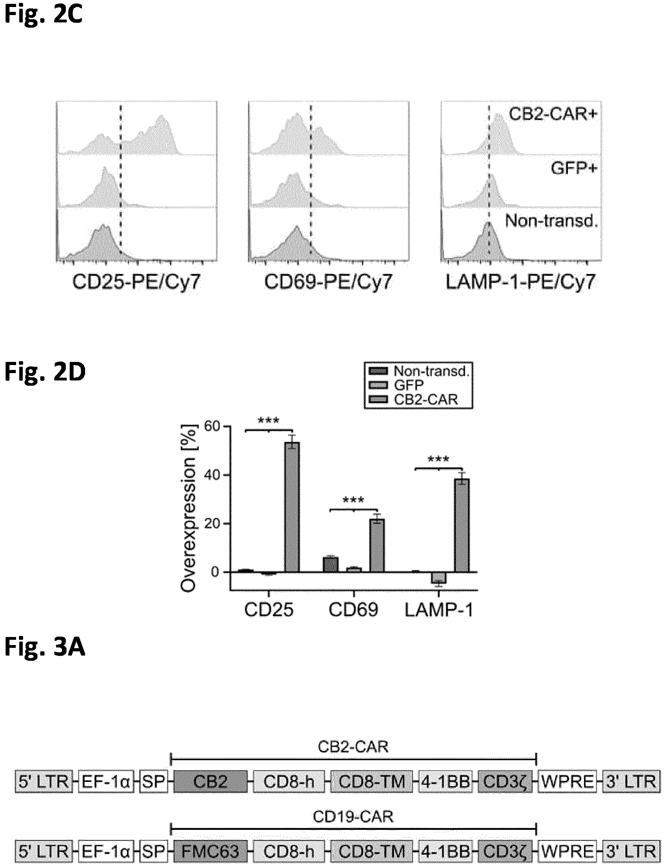

**Fig. 2C** Representative flow cytometry plots for the analysis of T cell activation markers. Different samples of T cells were co-cultured with SC-1 cells for 72 hours and stained with PE/Cyanine7-conjugated antibodies against the markers CD25, CD69, and LAMP-1 respectively and measured on a FACSCanto™ II Flow Cytometry System. Offset histograms of CB2-CAR-T cells and control T cells are shown for each marker. Dashed lines indicate thresholds for positive cells and were set according to T cells cultured without target cells.

**Fig. 2D** Quantification of T cell marker expression. Fractions of positive cells shown in C were quantified by subtracting the baseline expression of each marker (T cells only). Mean values ± SEM of n=3 replicates are shown, and significance of differences was assessed via 1-way ANOVA followed by Tukey's post-hoc test for each marker individually. Significance levels in all subfigures: n.s. - not significant; * - p<0.05; ** - p<0.01; *** - p<0.001.

**Fig. 3A** B cell lymphoma subtypes and antigen escape models are efficiently targeted by CB2-CAR-T cells. Domain structures of CB2-41BB-CAR and CD19-CAR. Abbreviations: LTR - long terminal repeat, EF-1$\alpha$ - human elongation factor-1 alpha promotor, SP - signal peptide, CD8-h - CD8 hinge, CD8-TM - CD8 transmembrane domain, 4-1BB - 4-1BB costimulatory domain, CD3$\zeta$ - CD3$\zeta$ signaling domain, WPRE - Woodchuck Hepatitis Virus Posttranscriptional Regulatory Element.

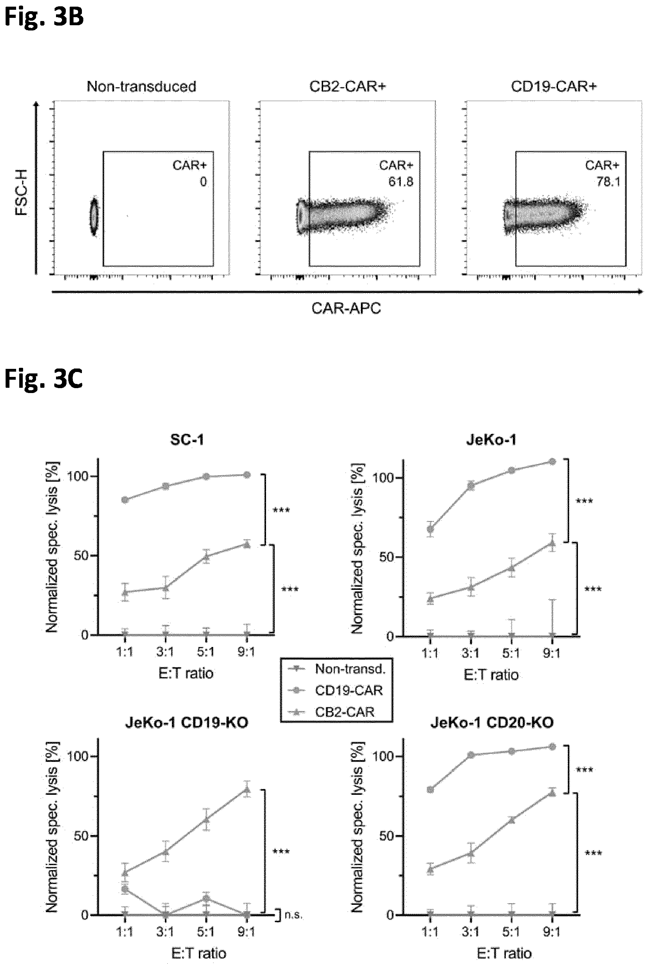

**Fig. 3B** Determination of transduction efficiencies of CB2-41BB-CAR and CD19-CAR on day 6 after transduction. Representative plots are shown. Cells were stained with MonoRab™ Rabbit Anti-Camelid VHH Cocktail iFluor 647 (CB2-41BB-CAR) or Recombinant Human CD19 Fc Chimera Alexa Fluor® 647 (CD19-CAR) and measured on a FACSCanto™ II Flow Cytometry System detecting CAR-APC fluorescence. Percentages of CAR+ T cells are indicated in the graphs.

**Fig. 3C** Luciferase-based cytotoxicity assays of different lymphoma cell lines co-cultured with CAR-T cells at different E:T ratios for 24 hours (SC-1) or 48 hours (JeKo-1, JeKo-1 CD19-KO, and JeKo-1 CD20-KO). Cytotoxicity was assessed by adding D-luciferin potassium salt and reading out relative light units with a CLARIOstar Plus plate reader. Data points were normalized to non-transduced T cells at the respective E:T ratio. Grand mean values ± pooled SEM of N=3, n=3 replicates are shown. Tested constructs are non-transduced (▼), CB2-41BB-CAR (▲), and CD19-CAR (●). Significance of differences was assessed via 2-way ANOVA followed by Tukey's post-hoc test (n.s. - not significant; * - p<0.05; ** - p<0.01; *** - p<0.001).

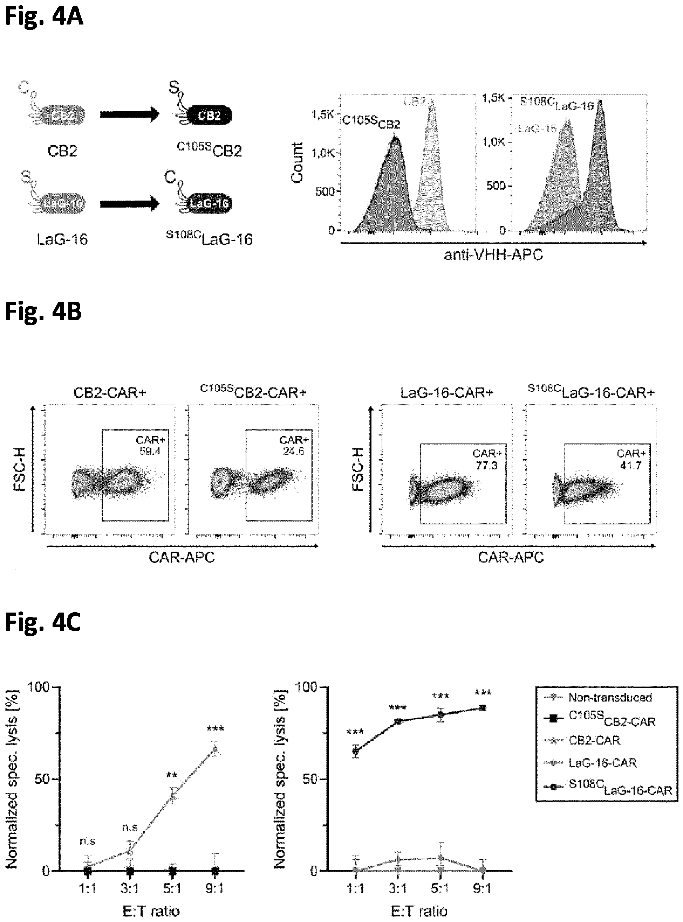

**Fig. 4A** Cysteine-engineered nanobodies redirect CAR-T cells to target B cell lymphoma. Illustration of investigated nanobodies and binding assays to SC-1 lymphoma. SC-1 cells were incubated with recombinant nanobodies and MonoRab™ Rabbit Anti-Camelid VHH Cocktail iFluor 647 and measured on a FACSCanto™ II Flow Cytometry System, detecting the anti-VHH-APC signal. Histograms for each nanobody and its mutant were overlayed to visualize differences in binding (grey histograms in the background represent secondary antibody-only controls). Representative plots of n=3 experiments are shown.

**Fig. 4B** Determination of transduction efficiencies of CB2-CAR- and LaG-16-CAR-T cells on day 6 after transduction. Representative plots are shown. Cells were stained with MonoRab™ Rabbit Anti-Camelid VHH Cocktail iFluor 647 and measured on a FACSCanto™ II Flow Cytometry System detecting CAR-APC fluorescence. Percentages of CAR+ T cells are indicated in the graphs.

**Fig. 4C** Luciferase-based cytotoxicity assays of SC-1 cells co-cultured with CAR-T cells at different E:T ratios for 24 hours. Cytotoxicity was assessed by adding D-luciferin potassium salt and reading out relative light units with a CLARIOstar Plus plate reader. Data points were normalized to non-transduced T cells at the respective E:T ratio. Grand mean values ± pooled SEM of N=3, n=3 replicates are shown. Tested constructs are non-transduced (▼), $^{C105S}$CB2-CAR (■), CB2-CAR (A), LaG-16-CAR (◆), and $^{S108C}$Lag-16-CAR (⬤). Significance of differences was assessed via 2-way ANOVA followed by Tukey's post-hoc test (n.s. - not significant; * - p<0.05; ** - p<0.01; *** - p<0.001).

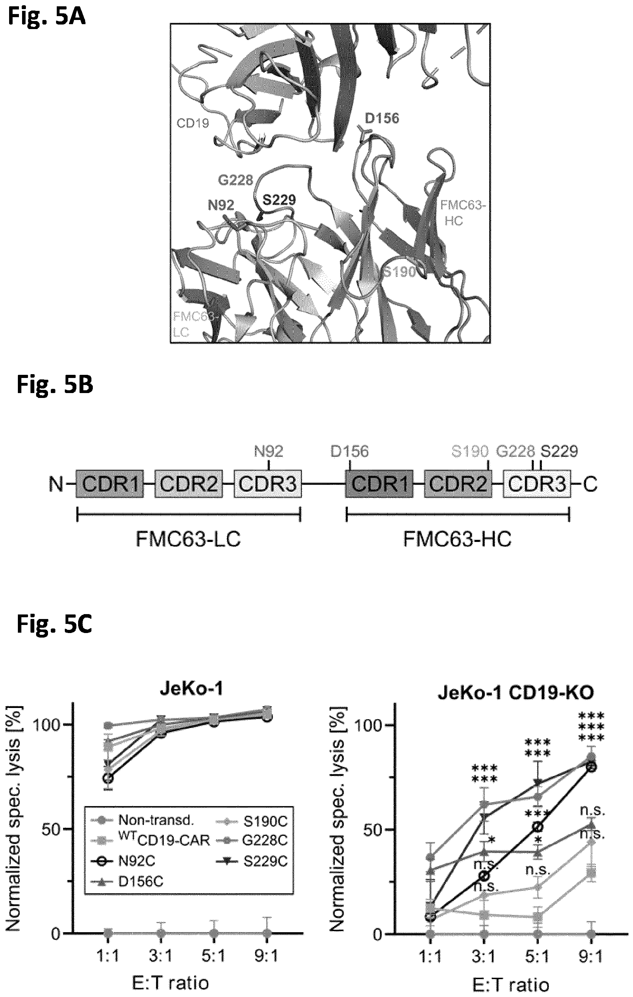

**Fig. 5A** Evaluation of Cytotoxic Activity of CD19-CAR Cysteine Mutants. Illustration of the mutated residues N92, D156, S190, G228, and S229 in the three-dimensional structure of FMC63 in complex with CD19 (PDB entry ID: 7URV).

**Fig. 5B** Schematic representation of the individual mutated residues in the linear sequence of FMC63 (LC - light chain, HC - heavy chain).

**Fig. 5C** Luciferase-based cytotoxicity assays of JeKo-1 lymphoma and JeKo-1 CD19-knockout co-cultured with CD19-CAR-T cell mutants at different E:T ratios for 48 hours. Cytotoxicity was assessed by adding D-luciferin potassium salt and reading out relative light units with a CLARIOstar Plus plate reader. Data points were normalized to non-transduced T cells at the respective E:T ratio. Mean values $\pm$ SEM of n=3 replicates are shown.

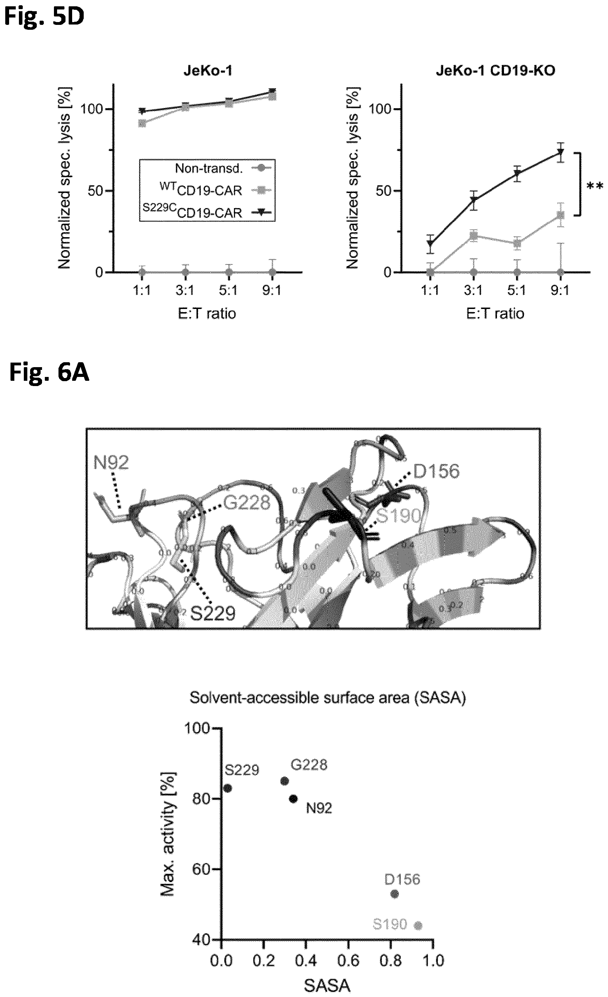

**Fig. 5D** Luciferase-based cytotoxicity assays of JeKo-1 lymphoma and JeKo-1 CD19-knockout co-cultured with $^{WT}$CD19-CAR- and $^{S229C}$CD19-CAR-T cells from three independent blood donors at different E:T ratios for 48 hours. Grand mean values $\pm$ pooled SEM of N=3, n=3 replicates are shown. Tested constructs are non-transduced (•), $^{WT}$CD19-CAR (■), $^{N92C}$CD19-CAR (○), $^{D156C}$CD19-CAR (▲), $^{S190C}$CD19-CAR (♦), $^{G228C}$CD19-CAR (⬤ ), and $^{S229C}$CD19-CAR (▼). Significance of differences was assessed via 2-way ANOVA followed by Tukey's post-hoc test (n.s. - not significant; * - p<0.05; ** - p<0.01; *** - p<0.001). Significance levels in comparison with $^{WT}$CD19-CAR are shown.

**Fig. 6A** Structural analysis of anti-CD19 scFv FMC63 to determine optimal solvent-accessible surface area (SASA). Structure was retrieved from the protein database PDB (Entry ID 7URV) and edited using PyMOL. SASA values were computed in PyMOL via the command "PyMOL>get_sasa_relative polymer". Coloring of the protein structure refers to SASA values (blue - high, grey - low). SASA of the candidate amino acids were plotted against maximum activity (effector-to-target ratio of 9:1) of respective T cells expressing the mutated scFvs in context of a CAR.

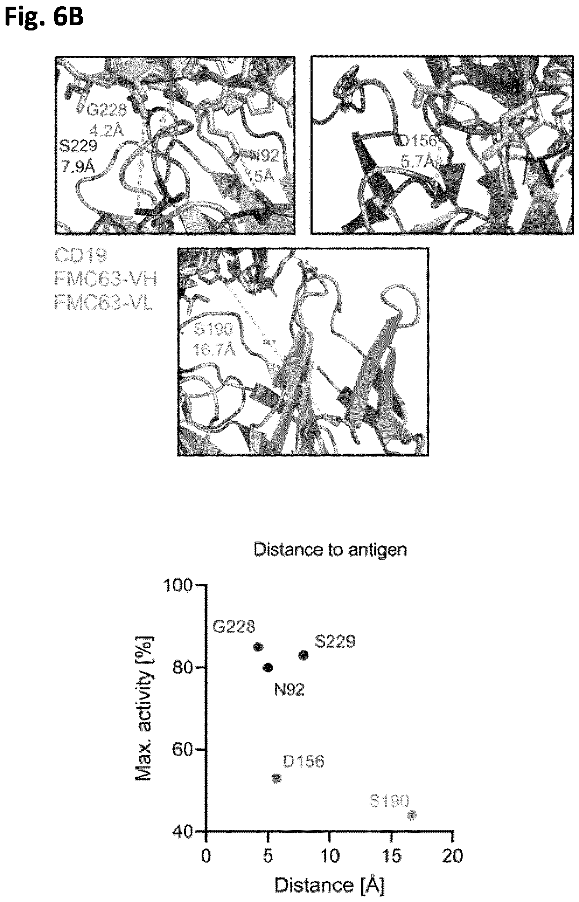

**Fig. 6B** Structural analysis of anti-CD19 scFv FMC63 to determine optimal distance to target antigen. (B) Distance values were computed in PyMOL using the measurement wizard tool. The distance of the respective side chain of the candidate amino acids with the closest atom of CD19 are shown and plotted against maximum activity (effector-to-target ratio of 9:1) of respective T cells expressing the mutated scFvs in context of a CAR.

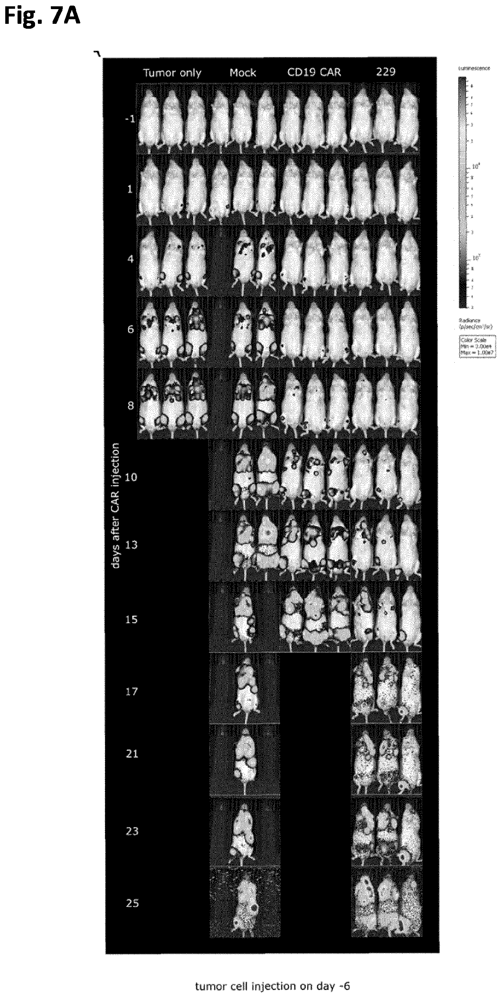

**Fig. 7A** *In vivo* assay with cysteine-engineered CD19-CAR-T cells. Mice were xenografted on day -6 with human B cell lymphoma cell lines JeKo-1 and JeKo-1 CD190-knockout at 1:1 ratio ($0.5\times10^6$ cells each; n=3 mice per group). At time points indicated, tumor development was measured via luciferase signal.

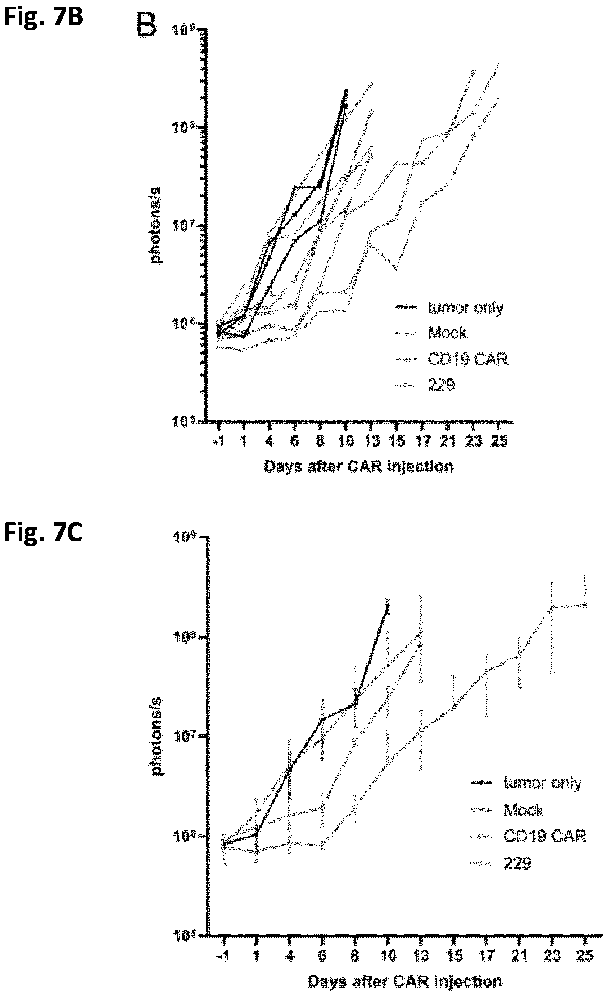

**Fig. 7B-C** Intensities in photons per second were quantified and plotted as individual curves per mouse (top panel) and average (lower panel). One mice of the mock group that showed unexpected long survival was excluded for the quantification.

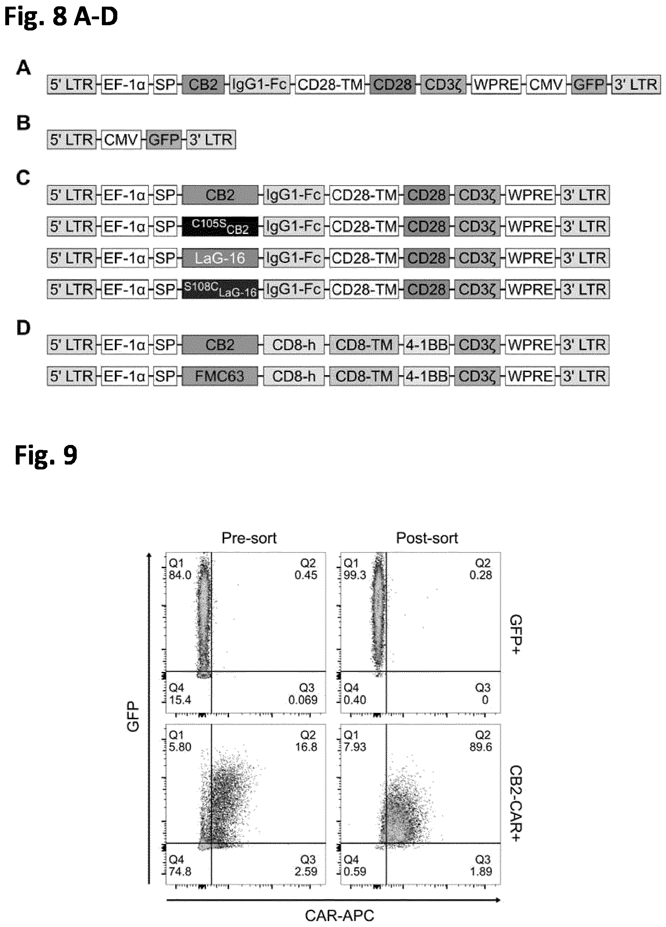

**Fig. 8A-D** Overview of lentiviral constructs. (A) CB2-CD28-CAR_GFP construct. (B) GFP control construct. (C) CD28-CAR constructs. (D) 41BB-CAR constructs. Abbreviations: LTR - long terminal repeat, EF-1$\alpha$ - human elongation factor-1 alpha promotor, SP - signal peptide, IgG1-Fc - human IgG1-Fc hinge, CD28-TM - CD28 transmembrane domain, CD28 - CD28 costimulatory domain, CD3$\zeta$ - CD3$\zeta$ signaling domain, WPRE - Woodchuck Hepatitis Virus Posttranscriptional Regulatory Element, CMV - human cytomegalovirus enhancer and promotor, GFP - green fluorescent protein, CD8-h - CD8 hinge, CD8-TM - CD8 transmembrane domain, 4-1BB - 4-1BB costimulatory domain.

**Fig. 9** Sorting efficiency of CB2-CD28-CAR_GFP and GFP control T cells. Cells were sorted using a FACSMelody™ Cell Sorter. Pre-sorting (left panels) and post-sorting (right panels) efficiencies were measured by staining with MonoRab™ Rabbit Anti-Camelid VHH Cocktail iFluor 647 and detecting antibody fluorescence (CAR-APC) and GFP fluorescence (GFP).

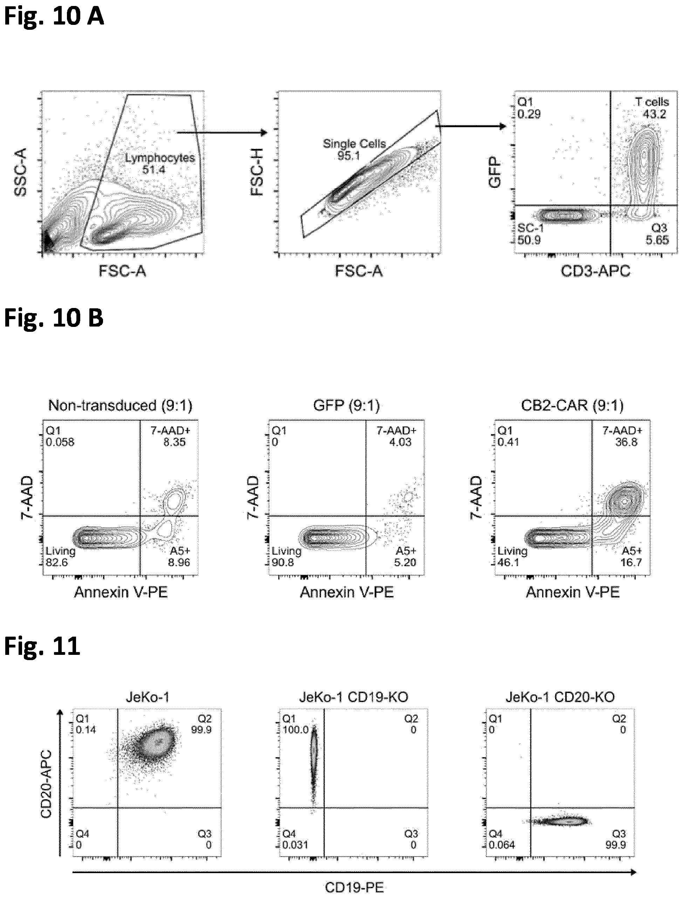

**Fig. 10A-B** Gating strategy for flow cytometry-based apoptosis assays. Co-cultured cells were stained with anti-CD3-APC antibody, Annexin V-PE, and 7-AAD Viability Staining Solution and measured on a FACSCanto™ II Flow Cytometry System. (A) Gating of single target cells (SC-1). Cells were gated for lymphocytes (left panel), single cells (middle panel), and SC-1 cells (GFP-/CD3-APC-; right panel). (B) Gating of apoptotic target cells. Single SC-1 cells (gated in A) were further gated for Annexin V-PE+/7-AAD- cells (early apoptotic cells) and Annexin V-PE+/7-AAD+ cells (late apoptotic cells). Exemplary plots for effector to target ratio of 9:1 are shown. Abbreviations: FSC - forward scatter, SSC - side scatter.

**Fig. 11** Knockout efficiencies of JeKo-1 cell lines. Cells were stained with anti-CD19-PE and anti-CD20-APC antibodies and measured on a FACSCanto™ II Flow Cytometry System. Gates were adjusted according to non-stained control (not shown). Left panel: JeKo-1, middle panel: JeKo-1 CD19-knockout, right panel: JeKo-1 CD20-knockout.

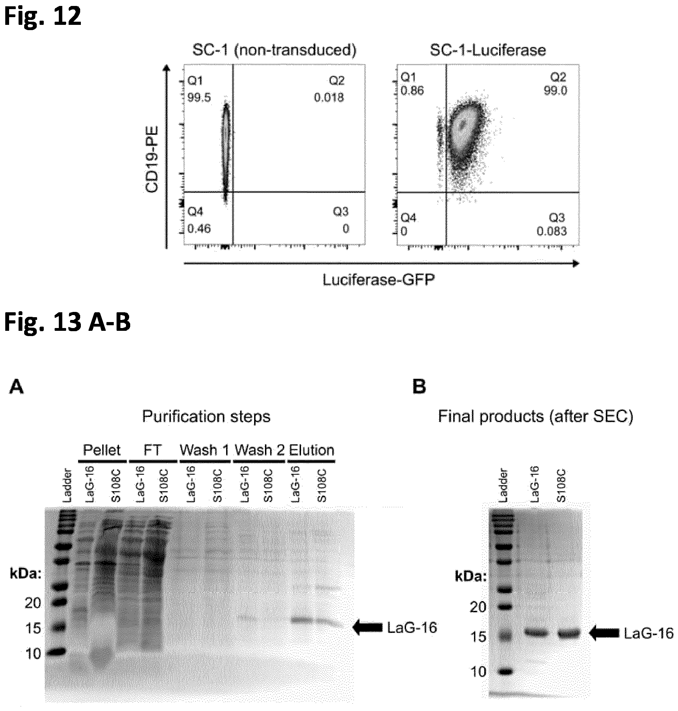

**Fig. 12:** Validation of firefly luciferase-expressing SC-1 cells. SC 1 cell line was transduced with lentivirus carrying firefly luciferase and GFP. Successfully transduced cells were single-cell sorted on a FACSMelody™ Cell Sorter and expanded. The verification of the clone used for subsequent assays is shown. Cells were stained with anti-CD19-PE antibody and measured on a FACSCanto™ II Flow Cytometry System. CD19 and Luciferase-GFP expression was assessed to confirm successful generation of SC-1_Luciferase cells.

**Fig. 13A-B:** SDS-PAGE gels of WT LaG-16 and S108CLaG-16 protein purification. Sodium dodecyl-sulfate poly-acrylamide gel electrophoresis (SDS-PAGE) separation demonstrating protein purity during the affinity purification of WT LaG-16 and S108CLaG-16 nanobodies (A) and the final protein product used for the different experiments after size exclusion chromatography (SEC) separation (B).

## Detailed description

The invention provides antigen-binding domains engineered to bind to antigens in an antigen-specific and/or antigen-independent, i.e., a thiol-dependent way, which is particularly advantageous for cancer treatment and imaging.

The present invention provides an antigen-binding domain that includes a heavy chain variable region (VH), and optionally a light chain variable region (VL). The antigen-binding domain is capable of binding to an antigen. The VH, and optionally the VL, include three complementarity-determining regions (CDRs). The CDRs are regions within the variable regions of the heavy and light chains that directly interact with the antigen. These regions are highly variable in sequence and are responsible for the specificity of the antigen-antibody interaction. Each variable region typically contains three CDRs. The skilled person is aware of the general structure of CDRs within an antigen-binding domain.

In the present invention, at least one CDR comprises at least one amino acid residue other than cysteine, which has a solvent-accessible surface area from 0.00 to 0.75 and is located at a distance to the antigen from 3 to 14 Å. The solvent-accessible surface area of an amino acid residue refers to the surface area of the residue that is accessible to a solvent molecule. The distance to the antigen refers to the distance between the amino acid residue and the antigen when the antigen-binding domain is bound to the antigen.

The solvent-accessible surface area of an amino acid residue refers to the surface area of the residue that is accessible to a solvent, such as water. Solvent-accessible surface area (SASA) is commonly known to the skilled person and the SASA quantifies the surface area of a biomolecule that is accessible to solvent molecules, typically water. SASA is defined as the surface area of a biomolecule that can be reached by a solvent molecule, which is often modeled as a sphere with a specific radius (commonly 1.4 Å, approximating the radius of a water molecule). The measurement of SASA was first introduced by Lee and Richards in 1971, and it is sometimes referred to as the Lee-Richards molecular surface. SASA is typically calculated using algorithms that simulate the rolling of a probe sphere over the surface of the biomolecule. The most common methods include the Shrake-Rupley Algorithm, the Linear Combination of Pairwise Overlaps (LCPO), or the Power Diagram Method. The skilled person is aware of suitable software to determine the SASA of proteins easily such as dr-sasa or GROMACS.

The distance to the antigen refers to the distance between the amino acid residue in the CDR and the antigen when the antigen-binding domain is bound to the antigen. The skilled person is aware of software such as PyMol and Chimera to determine the distance based on e.g. a PDB file.

The skilled person is aware that SASA and the distance can be measured according to a PDB filed of the antigen and the antigen-binding domain, which may be part of e.g. an antibody, antibody fragment, or nanobody, as demonstrated experimentally herein.

These factors are crucial in determining the optimal position for the thiol group containing amino acid substitution, ensuring that the substituted thiol group containing amino acid residue is in a position where it can effectively interact with the antigen and contribute to the stability of the binding interaction.

In other words, at least one amino acid residue is substituted to a thiol-group containing amino acid. Thiol-group containing amino acids, such as cysteine, have a sulfur atom in their side chain. This sulfur atom can form a disulfide bond with a sulfur atom of another cysteine residue.

The antigen-binding domain of the present invention can be part of an antibody molecule, or it can be a standalone domain that is used for antigen binding. The antigen-binding domain can be used in a variety of applications, including but not limited to, diagnostic assays, therapeutic treatments, and research tools.

In preferred embodiments, the solvent-accessible surface area of the at least one amino acid residue other than cysteine in the antigen-binding domain is from 0.00 to 0.70. More preferably, the solvent-accessible surface area is from 0.00 to 0.65. Even more preferably, the solvent-accessible surface area is from 0.00 to 0.60. Even more preferably, the solvent-accessible surface area is from 0.00 to 0.55. Most preferably, the solvent-accessible surface area is from 0.00 to 0.50.

The antigen-binding domain as described can have an amino acid residue that is substituted to thiol group containing amino acid and may be located at a distance to the antigen ranging from 3 to 13 Å. More preferably, the distance to the antigen can range from 3.5 to 12 Å. Even more preferably, the distance to the antigen can range from 4 to 11 Å. Even more preferably, the distance to the antigen can range from 4 to 10 Å. Even more preferably, the distance to the antigen can range from 4 to 9 Å. Most preferably, the distance to the antigen can range from 4 to 8 Å. Experimental evidence fur such distances is provided in Figure 6B showing that distances from 4 to 8 Å, such as 4, 5, 6, 7 or 8 Å, are most preferred.

The distance to the antigen refers to the distance between the amino acid residue and the antigen when the antigen-binding domain is bound to the antigen. The distance can be measured using various methods known in the art, such as X-ray crystallography or nuclear magnetic resonance spectroscopy.

In preferred embodiments, the solvent accessibility of the amino acid residue and the distance to the antigen are defined based on a structural model of the antigen and the antigen-binding domain. The structural model can be an X-ray structure, a cryo-electron microscopy (cryo-EM)-based model, or an artificial intelligence (AI)-generated model. Preferably, the structural model is an X-ray structure.

By using a structural approach, the invention provides a precise and accurate way to define the solvent accessibility of the amino acid residue and the distance to the antigen. This allows for the design of antigen-binding domains with enhanced stability and antigen-binding properties.

The Kd can be determined by various in vitro methods, which the skilled person is aware of. These methods include, for example, surface plasmon resonance (SPR), enzyme-linked immunosorbent assay (ELISA), fluorescence resonance energy transfer (FRET), Isothermal Titration Calorimetry (ITC), or MicroScale Thermophoresis (MST). SPR is preferred. Ideally, the Kd to the antigen is determinable, wherein the antigen-binding domain is part of an antibody, a Fab fragment, a single chain variable fragment (scFV), single domain antibody fragment, antigen binding domain fragment, a diabody or a combination thereof. These methods can provide accurate and reliable measurements of the Kd, which can be used to evaluate the binding interaction between the antigen-binding domain and the antigen. A thiol group containing amino acid is for example cysteine. Cysteine is a unique amino acid that contains a thiol group, which can form a disulfide bond with another cysteine residue. This can result in a more stable binding interaction between the antigen-binding domain and the antigen, which can enhance the effectiveness of the antigen-binding domain in recognizing and responding to the antigen.

The term 'thiol group containing amino acid' as used herein refers to natural and unnatural cysteine analogs. In general, the term 'thiol group containing amino acid' as used herein refers to any amino acid, natural or unnatural comprising a thiol group. A thiol group containing amino acid may be any cysteine analog or derivative containing a free thiol. Natural cysteines analogs include, but are not limited to, cysteamine, 3-mercapto propionic acid, cysteine sulfinic acid, and S-sulfocysteine. Unnatural cysteine analogs include, but are not limited to, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine. Most preferably, the thiol group containing amino acid is a cysteine. The thiol group containing amino acid is ideally selected from cysteine, cysteamine, 3-mercapto propionic acid, cysteine sulfinic acid, S-sulfocysteine, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine. Most preferably, the thiol group containing amino acid is a cysteine. In preferred embodiments, the antigen-binding domain is part of a larger antibody fragment. The antigen-binding domain can be part of a full-length antibody, Fab fragment, a single chain variable fragment (scFv), a single domain antibody fragment, an antigen-binding domain fragment, a diabody, or a combination thereof.

A Fab fragment is a portion of an antibody that includes the variable regions of the heavy and light chains, which together form the antigen-binding site, as well as part of the constant region of the heavy chain. A single chain variable fragment (scFv) is a fusion of the variable regions of the heavy and light chains, linked by a short peptide linker. A single domain antibody fragment is a single variable region, either from the heavy or light chain. An antigen-binding domain fragment can be a portion of an antibody that includes the antigen-binding site. A diabody is a small antibody fragment that consists of two scFvs linked together.

In another preferred embodiment of the invention, the antigen-binding domain is part of a larger molecular structure. The antigen-binding domain can be part of an antibody, a nanobody, a single chain variable fragment, or a Bi-specific T-cell engager.

An antibody is a large, Y-shaped protein. As known in the art, the antigen-binding domain is located at the tips of the "Y" and is responsible for the antibody's specificity for its target antigen. A nanobody is a fragment of a heavy chain antibody found in camelids and sharks. It consists of a single monomeric variable antibody domain and is one of the smallest fully functional antigen-binding fragments. A single chain variable fragment (scFv) is a fusion protein of the variable regions of the heavy and light chains of immunoglobulins, connected with a short linker peptide of about 10-25 amino acids. They are able to bind antigen with the same specificity as the parent antibody. A Bi-specific T-cell engager (BiTE) is a type of bispecific monoclonal antibody that is used as a medication to treat cancer. It functions by linking a T cell to a cancer cell, which results in the death of the cancer cell.

The antigen-binding domain may form part of an antibody, together with constant domains, a hinge region, and an Fc region. The constant domains provide the structural framework of the antibody and contribute to its effector functions.

The hinge region is a flexible part of the antibody that allows the antigen-binding domains to move relative to each other. The Fc region interacts with cell surface receptors and complement proteins to trigger immune responses.

Preferably, the antibody, comprising the antigen-binding domain according to the invention, is an IgG antibody. IgG antibodies are the most common type of antibody.

More preferably, the antibody is an IgG1 or IgG4 antibody. IgG1 and IgG4 antibodies are subclasses of IgG antibodies. IgG1 antibodies are the most abundant IgG subclass and are highly effective at activating complement proteins. IgG4 antibodies are less abundant but are unique in their ability to undergo Fab arm exchange, a process that results in bispecific antibodies.

Even more preferably, the antibody is an IgG1 antibody. IgG1 antibodies have been widely used in therapeutic applications due to their high effector function and good manufacturability. Ideally, the antigen-binding domain is additionally capable of binding in a thiol group-dependent manner to the target. This means that the binding of the antigen-binding domain to the target cell is influenced by the presence of a thiol group. The thiol group is part of the antigen-binding domain. The thiol group-dependent binding can be due to the formation of a disulfide bond between the thiol group of the antigen-binding domain and a cysteine residue in the antigen. Alternatively, the thiol group-dependent binding can be due to the formation of a disulfide bond between the thiol group of the antigen-binding domain and a cysteine residue on the target cell membrane, which is present due to the altered redox potential, in particular due to altered expression of enzymes regulating cell membrane redox system.

The ability of the antigen-binding domain to bind in a thiol group-dependent manner can enhance the stability of the antigen-[antigen-binding domain] complex, leading to improved antigen-binding properties. This is particularly beneficial in the development of therapeutic antibodies, where stability and binding affinity are critical for efficacy.

In preferred embodiments, one, two, three, or four amino acid residues in the antigen-binding domain are substituted to a thiol-group containing amino acid. More preferably, one, two, or three amino acid residues are substituted. Even more preferably, one or two amino acid residues are substituted. Most preferably, one amino acid residue is substituted to a thiol-group containing amino acid.

The number of amino acid residues that are substituted can be chosen based on the desired properties of the antigen-binding domain. For example, substituting a larger number of residues can lead to the formation of a new intra-disulfide bond and a greater increase in stability in addition to improved binding to the target cells. Therefore, the optimal number of substitutions can depend on the specific structure of the engineered antigen-binding domain and its requirements.

In preferred embodiments, the thiol-group containing amino acid substitution is located in the CDR3 of the VH of the antigen-binding domain. The CDR3 region is one of the three complementarity-determining regions in the variable region of the heavy chain (VH) of the antigen-binding domain. The CDR3 region typically plays a crucial role in antigen recognition and binding due to its central location in the antigen-binding site and its high degree of variability.

In preferred embodiments, the antigen-binding domain binds to any one antigen selected from CD276, CD19, GD2, CD20, CD22, CD33, CD123, CD38, BMCA, HER2, PMSA, mesothelin, NY-ESO-1 and EGFRvIII.

In preferred embodiments, the antigen-binding domain of the following anti-cancer antibodies:

| Name | Target; Format | 1st indication approved / reviewed |
|---|---|---|
| Datopotamab deruxtecan | TROP-2; Humanized IgG1 ADC | Non-small cell lung cancer |
| Zenocutuzumab | HER2, HER3; Humanized IgG1, Bispecific | NRG1+ non-small cell lung and pancreatic cancer |
| Zanidatamab | HER2; Humanized, Biparatopic bispecific | Biliary tract cancers |
| Linvoseltamab | BCMA, CD3; Human IgG4 | Multiple myeloma |
| Patritumab deruxtecan | HER3; Human IgG1 ADC | Non-small cell lung cancer |
| Tarlatamab | DLL, CD3; scFv-scFv-scFc bispecific | Small cell lung cancer |
| Zolbetuximab | Claudin 18.2; Chimeric IgG1 | Gastric or gastroesophageal junction adenocarcinoma |
| Odronextamab | CD20, CD3; Human IgG4, bispecific | Diffuse large B cell lymphoma, follicular lymphoma |
| Camrelizumab | PD-1; Human IgG4 | Hepatocellular carcinoma |
| Serplulimab | PD-1; Human IgG4 | Small cell lung cancer |
| Sugemalimab | PD-L1; Human IgG4 | Non-small cell lung cancer |

(continued)

| Name | Target; Format | 1st indication approved / reviewed |
|---|---|---|
| Hemophilia Cosibelimab | PD-L1; Human IgG1 | Squamous cell carcinoma |
| Trastuzumab duocarmazine | HER2; Humanized IgG1 ADC | Breast cancer |
| Sintilimab | PD-1; Human IgG4 | Non-small cell lung cancer |
| Elranatamab | BCMA, CD3; Humanized IgG2 | Multiple myeloma |
| Talquetamab | G protein-coupled receptor 5D, CD3; Humanized IgG4 bispecific | Multiple myeloma |
| Epcoritamab | CD20, CD3; Bispecific humanized IgG1 | Diffuse large B cell lymphoma |
| Glofitamab | CD20, CD3e; Bispecific 2+1 IgG1 CrossMab | Diffuse large B-cell lymphoma |
| Tislelizumab | PD-1; Humanized IgG4 | Esophageal squamous cell carcinoma |
| Toripalimab | PD-1; Humanized IgG4 | Nasopharyneal carcinoma, esophageal squamous cell carcinoma |
| Retifanlimab | PD-1; Humanized IgG4 | Merkel cell carcinoma |
| Mirvetuximab soravta nsi ne | Folate receptor alpha; Humanized IgG1 ADC | Ovarian cancer |
| Tremelimumab | CTLA-4; Human IgG2A | Antineoplastic; liver cancer |
| Teclistamab | BCMA, CD3; Humanized bispecific IgG4 | Multiple myeloma |
| Mosunetuzumab | CD20, CD3; Humanized bispecific IgG1 | Follicular lymphoma |
| Relatlimab | LAG-3; Human IgG4 | Melanoma |
| Tebentafusp | gp100, CD3; Bispecific immunoconjugate (TCR-scFv) | Metastatic uveal melanoma |
| Tisotumab vedotin | Tissue factor; Human IgG1 ADC | Cervical cancer |
| Amivantamab | EGFR, cMET; Human bispecific IgG1 | NSCLC w/ EGFR exon 20 insertion mutations |
| Loncastuximab tesirine | CD19; Humanized IgG1 ADC | Diffuse large B-cell lymphoma |
| Dostarlimab | PD-1; Humanized IgG4 | Endometrial cancer |
| Margetuximab | HER2; Chimeric IgG1 | HER2+ breast cancer |
| Naxitamab | GD2; Humanized IgG1 | High-risk neuroblastoma and refractory osteomedullary disease |
| Belantamab mafodotin | BCMA; Humanized IgG1 ADC | Multiple myeloma |
| Tafasitamab | CD19; Humanized IgG1 | Diffuse large B-cell lymphoma |
| Sacituzumab govitecan | TROP-2; Humanized IgG1 ADC | Triple-neg. breast cancer |
| Isatuximab | CD38; Chimeric IgG1 | Multiple myeloma |
| [fam]-trastuzumab deruxtecan | HER2; Humanized IgG1 ADC | HER2+ breast cancer |
| Polatuzumab vedotin | CD79b; Humanized IgG1 ADC | Diffuse large B-cell lymphoma |
| Cemiplimab | PD-1; Human IgG4 | Cutaneous squamous cell carcinoma |
| Moxetumomab pasudotox | CD22; Murine IgG1 dsFv immunotoxin | Hairy cell leukemia |
| Mogamulizumab | CCR4; Humanized IgG1 | Cutaneous T cell lymphoma |
| Durvalumab | PD-L1; Human IgG1 | Bladder cancer |

(continued)

| Name | Target; Format | 1st indication approved / reviewed |
|---|---|---|
| Inotuzumab ozogamicin | CD22; Humanized IgG4, ADC | Hematological malignancy |
| Avelumab | PD-L1; Human IgG1 | Merkel cell carcinoma |
| Atezolizumab | PD-L1; Humanized IgG1 | Bladder cancer |
| Olaratumab | PDGRFo; Human IgG1 | Soft tissue sarcoma |
| Daratumumab | CD38; Human IgG1 | Multiple myeloma |
| Elotuzumab | SLAMF7; Humanized IgG1 | Multiple myeloma |
| Necitumumab | EGFR; Human IgG1 | Non-small cell lung cancer |
| Dinutuximab | GD2; Chimeric IgG1 | Neuroblastoma |
| Nivolumab | PD1; Human IgG4 | Melanoma, non-small cell lung cancer |
| Blinatumomab | CD19, CD3; Murine bispecific tandem scFv | Acute lymphoblastic leukemia |
| Pembrolizumab | PD1; Humanized IgG4 | Melanoma |
| Ramucirumab | VEGFR2; Human IgG1 | Gastric cancer |
| Obinutuzumab | CD20; Humanized IgG1; Glycoengineered | Chronic lymphocytic leukemia |
| Ado-trastuzumab emtansine | HER2; Humanized IgG1, ADC | Breast cancer |
| Pertuzumab | HER2; Humanized IgG1 | Breast Cancer |
| Brentuximab vedotin | CD30; Chimeric IgG1, ADC | Hodgkin lymphoma, systemic anaplastic large cell lymphoma |
| Ipilimumab | CTLA-4; Human IgG1 | Metastatic melanoma |
| Ofatumumab | CD20; Human IgG1 | Chronic lymphocytic leukemia |
| Panitumumab | EGFR; Human IgG2 | Colorectal cancer |
| Bevacizumab | VEGF; Humanized IgG1 | Colorectal cancer |
| Cetuximab | EGFR; Chimeric IgG1 | Colorectal cancer |
| Tositumomab-I131 | CD20; Murine IgG2a | Non-Hodgkin lymphoma |
| Ibritumomab tiuxetan | CD20; Murine IgG1 | Non-Hodgkin lymphoma |
| Alemtuzumab | CD52; Humanized IgG1 | Chronic myeloid leukemia |
| Gemtuzumab ozogamicin | CD33; Humanized IgG4, ADC | Acute myeloid leukemia |
| Trastuzumab | HER2; Humanized IgG1 | Breast cancer |
| Rituximab | CD20; Chimeric IgG1 | Non-Hodgkin lymphoma |
| Edrecolomab | EpCAM; Murine IgG2a | Colorectal cancer |

These antigens are associated with cancers, and are therefore important targets for therapeutic antibodies. For example, CD19 is a protein expressed on the surface of B cells and is a target for therapies against B cell malignancies, such as lymphoma. GD2 is a ganglioside found on the surface of cells and is a target for therapies against triple negative breast cancer, neuroblastoma and other tumors. CD20 is a protein found on the surface of B cells and is a target for therapies against B cell lymphomas and leukemia. CD22, CD33, CD123, and CD38 are also proteins found on the surface of certain immune cells and are targets for therapies against various hematological malignancies. BMCA, HER2, PMSA, mesothelin, NY-ESO-1 and EGFRvIII are proteins associated with various types of solid tumors and are targets for therapies against these tumors.

In preferred embodiments, the antigen-binding domain binds to CD19. More preferably, the CD19 antigen-binding domain is scFV FMC63, comprising at least one substitution according to the claims. scFMC63 is a single-chain variable fragment that is known to bind to CD19 with high affinity.

In another preferred embodiment, the CD19 antigen-binding domain comprises, and preferably consists of, a sequence having at least 70% identity to SEQ ID NO: 1, which represents the amino acid sequence of scFV FMC63. More preferably, the sequence has at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 1.

As used herein, sequence identity refers to the identity of amino acids between two sequences. It is calculated by aligning the sequences and determining the percentage of positions at which the same amino acid residue is present in both sequences. The skilled person is aware of online tools to align amino acid sequences and obtain an identity percentage such as Expasy.

In preferred embodiments, the CD19 antigen-binding domain has the sequence of SEQ ID NO:1, and the residue at position 229 of SEQ ID NO: 1 is substituted to a thiol-group containing amino acid.

Ideally, the CD19 antigen-binding domain has the sequence of SEQ ID NO:1, and the residue at position 228 of SEQ ID NO:1 is substituted to a thiol-group containing amino acid.

In particular, the CD19 antigen-binding domain has the sequence of SEQ ID NO:1, and the residue at position 92 of SEQ ID NO:1 is substituted to a thiol-group containing amino acid. These specific substitutions are chosen based on the structure and function of the antigen-binding domain.

In preferred embodiments, the antigen-binding domain is CB2. CB2 is a naturally occurring nanobody discovered in previous work, which has a single cysteine residue present in CDR3.

More preferably, the CB2 antigen-binding domain comprises, and preferably consists of, a sequence having at least 70% identity to SEQ ID NO: 5, which represents the amino acid sequence of a known CB2-binding domain. More preferably, the sequence has at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 5.

In preferred embodiments, the antigen-binding domain is LAG-16. LAG-16 is a known single domain nanobody that binds a specific target, the green fluorescent protein (GFP). More preferably, the LAG-16 antigen-binding domain comprises, and preferably consists of, a sequence having at least 70% identity to SEQ ID NO: 6, which represents the amino acid sequence of a known LAG-16 binding domain. More preferably, the sequence has at least 75%, 80%, 81%, 82%, 83%, 84%, 85%, 86%, 87%, 88%, 89%, 90%, 91%, 92%, 93%, 94%, 95%, 96%, 97%, 98%, 99%, or even 100% identity to SEQ ID NO: 6. Preferably, the amino acid at position 108 is substituted to a thiol-group containing amino acid with respect to SEQ ID NO:6.

Preferably, the antigen-binding domain additionally binds to the target cell in an antigen-independent manner. This means that the antigen-binding domain can bind to the target cell even in the absence of the specific antigen.

Ideally, the target cell is a cancer cell. Cancer cells often do express specific antigens, which are not expressed on normal cells. These cancer-specific antigens can be targeted by antibodies and other antigen-binding molecules for the purpose of cancer diagnosis or therapy.

It is preferred that the binding of the antigen-binding domain to the target cell is detectable by flow cytometry on antigen positive and antigen negative cells. Flow cytometry is a technique used to detect and measure physical and chemical characteristics of a population of cells. In this case, it can be used to detect the binding of the antigen-binding domain to the target cells.

In preferred embodiments, the antigen-independent binding of the antigen-binding domain to the target cell involves the formation of at least one bond between at least one thiol-group containing amino acid residue within at least one CDR of the antigen-binding domain and at least one cysteine residue present in at least one surface protein of a cell.

The formation of a disulfide bond between the antigen-binding domain and the target cell can provide a mechanism for antigen-independent binding. This can be particularly beneficial in situations where the specific antigen is not present, or is present at low levels, on the surface of the target cell. This mechanism often detected in various cancers is called antigen escape. The antigen-independent binding can also enhance the overall binding affinity of the antigen-binding domain for the target cell, potentially leading to improved therapeutic efficacy.

In preferred embodiments, the antigen-binding domain is for use in a method of treating cancer. Cancer is a group of diseases characterized by the uncontrolled growth and spread of abnormal cells, which the skilled person is aware of.

The antigen-binding domain can be used to target cancer cells for destruction by the immune system. The antigen-binding domain can bind to specific antigens present on the surface of cancer cells, marking these cells for destruction. The antigen-binding domain can also recruit immune cells to the site of the cancer, enhancing the immune response against the cancer cells.

The antigen-binding domain can be used in a variety of cancer treatment methods, including but not limited to, immunotherapy, targeted therapy, and antibody-drug conjugate therapy. The specific method of treatment can be chosen based on the type of cancer and the stage of the disease.

In preferred embodiments, the method of treatment using the antigen-binding domain inhibits cancer growth, metastasis formation, and/or prolongs survival. Cancer growth refers to the uncontrolled proliferation of cancer cells, leading to the formation and growth of a tumor. The antigen-binding domain can inhibit cancer growth by binding to specific antigens on the surface of cancer cells, marking these cells for destruction by the immune system.

Metastasis formation refers to the process by which cancer cells spread from the primary tumor to other parts of the body. The antigen-binding domain can inhibit metastasis formation by targeting and eliminating circulating tumor cells, which are cancer cells that have detached from the primary tumor and entered the bloodstream or lymphatic system.

Prolonging survival refers to extending the lifespan of a patient with cancer. The antigen-binding domain can prolong survival by effectively controlling the growth and spread of the cancer, thereby slowing the progression of the disease.

In preferred embodiments, the method of treatment using the antigen-binding domain is applied to a cancer that comprises a subset of cancer cells characterized by a reduced expression of the antigen. This situation can occur in various types of cancer, where a subset of the cancer cells may downregulate or completely lose the expression of the antigen that is targeted by the antigen-binding domain. This phenomenon, known as antigen escape, can lead to the survival and proliferation of these antigen-negative cancer cells.

The antigen-binding domain of the present invention ideally bind to the target cell in an antigen-independent manner, meaning that it can bind to the target cell even in the absence of the specific antigen. This can be particularly beneficial in situations where the cancer cells have undergone antigen escape. The antigen-independent binding can enhance the overall binding affinity of the antigen-binding domain for the target cell, potentially leading to improved therapeutic efficacy.

In preferred embodiments, the method of treatment using the antigen-binding domain is applied to a cancer that comprises a subset of cancer cells that do not express the antigen targeted by the antigen-binding domain. In this context, it is specified that the antigen-negative subset constitutes at least 0.001% of the total cancer cell population, do not express the antigen targeted by the CAR, preferably at least 0.005%, more preferably at least 0.01%, even more preferably at least 0.05%, even more preferably at least 0.1%, even more preferably at least 0.5%. Ideally, the cancer cells, constituting at most 50% of the total cancer cell population, do not express the antigen targeted by the CAR. This percentage can be determined by flow cytometry (FACS).

In another preferred embodiment, the method of treatment using the antigen-binding domain is applied to an antigen-positive cancer to prevent antigen escape. In other words, the antigen-positive cancer cells have the risk of undergoing antigen escape. The antigen-binding domain can also be used in a method of treatment of a cancer that has the risk to comprise a subset of cancer cells that do not express the antigen targeted by the CAR. Antigen escape often occurs after the treatment starts in order to escape the treatment. The advantage in reducing antigen escape is therefore also present for cancers with 0% antigen-negative cells.

The presence or absence of the targeted antigen can be detectable, preferably by FACS, Western blot, RNA-seq or mass spectrometry. These techniques allow for the detection and quantification of the antigen at the protein level (FACS, Western blot, mass spectrometry) or at the mRNA level (RNA-seq), providing valuable information about the expression of the antigen in the cancer cells.

In preferred embodiments, the method of treatment using the antigen-binding domain is applied to a cancer cell that has at least one enzyme that catalyses the formation and/or breakage of disulfide bonds between cysteine residues, compared to the expression level of the same enzyme in a non-cancerous cell of the same cell type. It is preferred, the targeted cancer cell exhibits at least a 1.6-fold, more preferably at least 1.7-fold, more preferably 1.8-fold, more preferably 1.9-fold, and even more preferably 2-fold, increase in the expression level of at least one enzyme that catalyses the formation and/or breakage of disulfide bonds between cysteine residues. An increase in the expression level of these enzymes in cancer cells can influence the redox state of the cells and contribute to the survival and proliferation of the cancer cells.

The expression level of the enzyme can be detectable by using mass spectrometry, RNA-seq or immunodetection methods, preferably a Western blot. The preferred method is mass spectrometry. These techniques allow for the detection and quantification of the enzyme at in the cancer cells. The enzyme is preferably selected from protein disulfide isomerase family or thioredoxin protein class. Protein disulfide isomerases (PDI) are enzymes that catalyzes the formation and rearrangement of disulfide bonds. Thioredoxin are enzymes that are known to reduce disulfide bonds in proteins. Exemplary PDIs are PDIA1 (P4HB, PDIA2 (PDIp), PDIA3 (ERp57, GRP58), PDIA4 (ERp72), PDIA5, PDIA6 (P5, TXNDC7), PDIR (PDIA5), ERp44, ERp29, TMX (TXNDC), and ERdj5. Exemplary thioredoxins are TXN (Thioredoxin), TXN2 (Thioredoxin 2), TXNDC1, TXNDC2, TXNDC3, TXNDC4, TXNDC5, TXNDC6, TXNDC8, TXNDC10, TXNDC11, TXNDC13, TXNDC14, TXNDC15, TXNDC16, TXNL1, and TXNL3.

In preferred embodiments, the method of treatment using the antigen-binding domain is applied to a cancer cell that expresses CD19, CD276, or GD2 on its surface. CD19 is a protein expressed on the surface of B cells and is a target for therapies against B cell malignancies. GD2 is a ganglioside found on the surface of cells and is a target for therapies against triple negative breast cancer, neuroblastoma and other tumours. CD276, also known as B7-H3, is a cell surface protein that belongs to the B7 family of immune checkpoint molecules. It is overexpressed in various types of cancers, including breast, lung, prostate, sarcoma, and brain tumors, and is associated with tumor progression, poor prognosis, and resistance to therapy.

The antigen-binding domain can bind to CD19, GD2, or CD276 with high specificity, e.g., marking these cells for destruction by the immune system. The antigen-binding domain can also recruit immune cells to the site of the cancer, enhancing the immune response against the cancer cells.

In preferred embodiments, the method of treatment using the antigen-binding domain is applied to a hematological cancer or a solid tumor. The skilled person is aware of hematological cancer or a solid tumor and how to diagnose them.

The antigen-binding domain can bind to antigens present on the surface of the cancer cells and ideally additionally bind in a thiol-dependent manner, marking these cells for destruction by the immune system. The antigen-binding domain can also recruit immune cells to the site of the cancer, enhancing the immune response against the cancer cells.

More preferably, the solid tumor is breast cancer, colon cancer, lung cancer, skin cancer, brain cancer, head and neck cancer, soft tissue cancer, gastrointestinal cancer, genitourinary and gynecologic cancer, musculoskeletal cancer, sarcoma or endocrine cancer. These are all common types of solid tumors that can benefit from treatment with the antigen-binding domain.

In particular, the solid tumor can be a breast cancer or a brain cancer. Breast cancer is a cancer that forms in the cells of the breasts, and brain cancer is a cancer that forms in the cells of the brain. Both of these cancers can be difficult to treat, and the antigen-binding domain can provide a new therapeutic option for these cancers.

In preferred embodiments, the method of treatment using the antigen-binding domain is applied to a hematological cancer, specifically a lymphoma or a leukemia. There are several

types of lymphoma, including Hodgkin's lymphoma and non-Hodgkin's lymphoma. There are several types of leukemia, including acute lymphoblastic leukemia, acute myeloid leukemia, chronic lymphocytic leukemia, and chronic myeloid leukemia.

In preferred embodiments, the method of treatment using the antigen-binding domain is applied to a specific type of lymphoma, such as Non-Hodgkin Lymphoma, Hodgkin lymphoma, Burkitt Lymphoma, multiple myeloma, or follicular lymphoma.

In preferred embodiments, the method of treatment using the antigen-binding domain is applied to a specific type of leukemia, such as Lymphoblastic Leukemia, Acute Myeloid Leukemia, Chronic Lymphocytic Leukemia, Chronic Myeloid Leukemia, Chronic Myelomonocytic Leukemia, Hairy Cell Leukemia, Prolymphocytic Leukemia, Myelodysplastic Syndromes, or Mixed-Phenotype Acute Leukemia.

In preferred embodiments, the method of treatment using the antigen-binding domain is applied to a specific type of breast cancer, such as triple-negative breast cancer, HER2-positive breast cancer, or hormone receptor-positive breast cancer.

In preferred embodiments, the method of treatment using the antigen-binding domain is applied to a specific type of solid tumor, such as melanoma, osteosarcoma, Ewing sarcoma, retinoblastoma, pediatric rhabdomyosarcoma, sarcoma, or small cell lung cancer.

Furthermore, the antigen binding domain may be used in a method of treating a specific type of brain cancer, such as glioblastoma or neuroblastoma.

Moreover, the antigen-binding domain may be used for imaging of specific cell populations with altered redox conditions or in an immunomodulating therapy. As known to the skilled person, immunomodulating therapies are treatments designed to alter the immune system's response to achieve a therapeutic effect. These therapies can either enhance or suppress the immune response, depending on the condition being treated. In one aspect, the immunomodulating therapy enhances the immune response, often used in cancer treatment to help the immune system target and destroy cancer cells.

The antigen-binding domain can bind to antigens present on the surface of cancer cells with altered redox conditions, and thereby distinguish them from their healthy counterparts. The antigen-binding domain may be used for imaging agents and marking abnormal cells or tissues in patients through non-invasive molecular imaging techniques such as Magnetic resonance imaging (MRI), positron emission tomography (PET), and single-photon emission computerized tomography (SPECT).

Moreover, the antigen-binding domain can bind to antigens present on the surface of cancer cells with altered redox conditions for molecular investigation for therapy compatibility *in vitro* of patient-derived tissues and cell lines using the above mentioned techniques as well as Enzyme-linked immunosorbent assay (ELISA), Enzyme-linked immunospot (ELISPOT), Western blot (WB), Immunoprecipitation (IP), Chromatin immunoprecipitation (ChIP), Immunohistochemistry (IHC), Immunocytochemistry (ICC), and Flow cytometry (FACS).

The present invention also provides a method for generating an antigen-binding domain that includes a heavy chain variable region and, optionally, a light chain variable region. The method comprises the following steps:

a) Selecting at least one amino acid residue suitable for cysteine substitution. This selected amino acid residue is located within a complementarity-determining region (CDR) of the heavy chain variable region (VH) or the light chain variable region (VL). The selected residue has a solvent-accessible surface area from 0.00 to 0.75 and is located at a distance to the antigen from 3 to 14 Å. The solvent-accessible surface area and the distance to the antigen are ideally defined based on a structural model of the antigen and the antigen-binding domain.

b) Mutating the selected residue to a thiol-group containing amino acid. This mutation can be introduced using various methods known in the art, such as site-directed mutagenesis.

c) Ideally, testing the binding of the thiol-group containing amino acid engineered antigen-binding domain to the antigen. This can be done using various methods known in the art, such as surface plasmon resonance (SPR), enzyme-linked immunosorbent assay (ELISA), fluorescence resonance energy transfer (FRET), Isothermal Titration Calorimetry (ITC), MicroScale Thermophoresis (MST).

The method of the invention allows for the generation of antigen-binding domains with enhanced antigen-binding properties. This can be particularly beneficial in the development of therapeutic antibodies, where stability, binding affinity, and/or effector function are critical for efficacy.

The invention is also described by the following embodiments:

1. An antigen-binding domain comprising a heavy chain variable region (VH), and optionally a light chain variable region (VL); wherein the antigen binding domain is capable of binding to an antigen, characterised in that the VH, and optionally the VL, comprise three complementarity-determining regions (CDRs), wherein at least one CDR comprises at least one amino acid residue other than cysteine, which has a solvent-accessible surface area from 0.00 to 0.75 and is located at a distance to the antigen from 3 to 14 Å,
and wherein said at least one amino acid residue is substituted to a thiol-group containing amino acid.

2. The antigen-binding domain of embodiment 1, wherein the solvent-accessible surface area is from 0.00 to 0.70, more preferably area is from 0.00 to 0.65, even more preferably is from 0.00 to 0.60, even more preferably is from 0.00 to 0.55, and most preferably is from 0.00 to 0.50.

3. The antigen-binding domain of any one of the preceding embodiments, wherein the residue for cysteine substitution is located at the distance to the antigen of 3-13 Å, more preferably of 3.5-12 Å, even more preferably of 4-11 Å, even more preferably of 4-10 Å, even more preferably of 4-9 Å, and most preferably of 4-8 Å.

4. The antigen-binding domain of any one of the preceding embodiments, wherein the solvent accessibility and the distance to the antigen are defined based on a structural model of the antigen and the antigen binding domain, wherein the structural model is an X-ray structure, a cryo-EM-based model, or an AI-generated model, preferably an X-ray structure.

5. The antigen-binding domain of any one of the preceding embodiments, wherein the antigen-binding domain binds the antigen with at least 1 mM $K_d$, wherein the $K_d$ is determinable by surface plasmon resonance (SPR), enzyme-linked immunosorbent assay (ELISA), Isothermal Titration Calorimetry (ITC), or MicroScale Thermophoresis (MST).

6. The antigen-binding domain of any one of the preceding embodiments, wherein the thiol-group containing amino acid is selected from cysteine, cysteamine, 3-mercapto propionic acid, cysteine sulfinic acid, S-sulfocysteine, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine, preferably wherein the thiol-group containing amino acid is cysteine.

7. The antigen-binding domain of any one of the preceding embodiments, wherein the antigen binding domain is a Fab fragment, a single chain variable fragment (scFV), single domain antibody fragment, antigen binding domain fragment, a diabody or a combination thereof.

8. The antigen-binding domain of any one of the preceding embodiments, wherein the antigen binding domain is part of an antibody, a nanobody, a single chain variable fragment, or a Bi-specific T-cell engager.

9. The antigen-binding domain of any one of embodiments 1 to 7, wherein the antigen binding domain forms an antibody together with constant domains, a hinge region and a Fc region, preferably wherein the antibody is an IgG antibody, more preferably wherein the antibody is an IgG1 or IgG4 antibody, even more preferably wherein the antibody is an IgG1 antibody.

10. The antigen-binding domain of embodiment 9, wherein the Fc region of the antibody is altered to improve antibody-dependent cytotoxicity.

11. The antigen-binding domain of embodiment 9, wherein the Fc region of the antibody comprises one or more substituted amino acids, preferably wherein the Fc region comprises one or more of the amino acids substitutions K322A, L234A, and L235A, preferably at least two of these substitutions, even more preferably all three of these substitutions.

12. The antigen-binding domain of any one of embodiments 9-11, wherein the Fc region of the antibody comprises a modified glycosylation pattern.

13. The antigen-binding domain of any one of embodiments 9-12, wherein the Fc region of the antibody is afucosylated.

14. The antigen-binding domain of any one of embodiments 9-13, wherein the Fc region of the antibody is switched, preferably from IgG4 to IgG1.

15. The antigen-binding domain of any one of the preceding embodiments, wherein the antigen-binding domain binds to a target cell in an antigen-dependent manner, preferably wherein the antigen-binding domain additionally binds to the target in an antigen-independent manner, more preferably wherein the target cell is a cancer cell, even more preferably wherein the binding is detectable by flow cytometry on antigen positive and antigen negative cells.

16. The antigen-binding domain of embodiment 15, wherein the antigen-independent binding comprises formation of at least one bond between at least one thiol group containing amino acid residue within at least one CDR of the antigen-binding domain and at least one cysteine residue present in at least one surface protein of a cell.

17. The antigen-binding domain of any one of the preceding embodiments, wherein one, two, three or four amino acid residue(s) are substituted to a thiol-group containing amino acid, preferably one, two or three, more preferably one or two, and even more preferably wherein one amino acid residue is substituted to a thiol-group containing amino acid.

18. The antigen-binding domain of any one of the preceding embodiments, wherein the thiol-group containing amino acid substitution is in the CDR3 of the VH of the antigen-binding domain.

19. The antigen-binding domain of any one of the preceding embodiments, wherein the antigen-binding domain binds any one antigen selected from CD19, GD2, CD20, CD22, CD33, CD123, CD38, CD276, BMCA, HER2, PMSA, mesothelin, NY-ESO-1 and EGFRvIII.

20. The antigen-binding domain of the embodiment 19, wherein the antigen-binding domain binds CD19, more preferably wherein the CD19 antigen-binding domain is scFV FMC63 or wherein the CD19 antigen-binding domain comprises and preferably consists of a sequence having at least 70% identity to SEQ ID NO: 1 [scFV FMC63], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 1; wherein at least one amino acid residue in at least one CDR is substituted to the thiol-group containing amino acid, preferably wherein the amino acid is at position 229, 228 and/or 92.

21. The antigen-binding domain of the embodiment 19, wherein the CD19 antigen-binding domain has the sequence of SEQ ID NO:1 and wherein the residue at the position 229 of SEQ ID NO:1 is substituted to the thiol-group containing amino acid; and/or

wherein the CD19 antigen-binding domain has the sequence of SEQ ID NO:1 and wherein the residue at the position 228 of SEQ ID NO:1 is substituted to the thiol-group containing amino acid; and/or

and/or wherein the CD19 antigen-binding domain has the sequence of SEQ ID NO:1 and wherein the residue at the position 92 of SEQ ID NO:1 is substituted to the thiol-group containing amino acid.

22. The antigen-binding domain of the embodiment 19, wherein the CD19 antigen-binding domain has the sequence of SEQ ID NO:1 and wherein the residue at the position 229 of SEQ ID NO:1 is substituted to the thiol-group containing amino acid; or

wherein the CD19 antigen-binding domain has the sequence of SEQ ID NO:1 and wherein the residue at the position 228 of SEQ ID NO:1 is substituted to the thiol-group containing amino acid;

or wherein the CD19 antigen-binding domain has the sequence of SEQ ID NO:1 and wherein the residue at the position 92 of SEQ ID NO:1 is substituted to the thiol-group containing amino acid.

23. The antigen-binding domain of any one of the embodiments 1-18, wherein the antigen-binding domain is CB2, more preferably wherein the antigen-binding comprises and preferably consists of a sequence having at least 70% identity to SEQ ID NO: 5 [CB2 WT], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 5.

24. The antigen-binding domain of any one of the embodiments 1-18, wherein the antigen-binding domain is LAG-16, more preferably wherein the antigen-binding comprises and preferably consists of a sequence having at least 70% identity to SEQ ID NO: 6 [LAG16 WT], preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 16; and wherein at least one amino acid residue in at least one CDR is substituted to the thiol-group containing amino acid, preferably wherein an amino acid at the position 108 substituted to the thiol-group containing amino acid with respect to SEQ ID NO:6.

25. The antigen-binding domain of any one of the preceding embodiments, wherein the antigen-binding domain binds to a target cell in an antigen-dependent manner, preferably wherein the antigen-binding domain additionally binds to the target cell in an antigen-independent manner, more preferably wherein the target cell is a cancer cell, even more preferably wherein the binding is detectable by flow cytometry on antigen positive and antigen negative cells.

26. The antigen-binding domain of embodiment 25, wherein the antigen-independent binding comprises formation of at least one bond between at least one thiol-group containing amino acid residue within at least one CDR of the antigen-binding domain and at least one cysteine residue present in at least one surface protein of a cell.

27. The antigen-binding domain according to any of embodiments 1-26 **for use** in a method of treating a cancer.

28. The antigen-binding domain for use in the method of treatment according to embodiment 27, wherein the method of treatment inhibits cancer growth, metastasis formation and/or prolongs survival.

29. The antigen-binding domain for use in the method of treatment according to any of the embodiments 27-28, wherein the cancer comprises a subset of cancer cells that is characterized in a reduced expression of the antigen, in particular wherein (i) the cancer cells have undergone an antigen escape; or (ii) wherein the cancer has a risk of undergoing antigen-escape.

30. The antigen-binding domain for use in the method of treatment according to embodiment 29, wherein the cancer comprises a subset of cancer cells that do not express the antigen targeted by the antigen-binding domain, and wherein:

said antigen-negative subset is at least 0.5% of the total cancer cell population as determinable by FACS; preferably the presence or absence of the targeted antigen is detectable by FACS, Western blot, RNA-seq or mass spectrometry.

31. The antigen-binding domain for use in the method of treatment according to any of the embodiments 27-30, wherein:

the cancer cell targeted in said method of treatment has at least a 2-fold increase in the expression level of at least one enzyme that catalyses the formation and/or breakage of disulfide bonds between cysteine residues, compared to the expression level of said enzyme in a non-cancerous cell of the same cell type; preferably wherein the expression level of the enzyme is detectable by using mass spectrometry, RNA-seq or immunodetection methods, preferably a Western blot; and the enzyme is preferably selected from a protein disulfide isomerase (PDI) or a thioredoxin protein.

32. The antigen-binding domain for use in the method of treatment according to any one of the embodiments 27-31 wherein the cancer cell targeted in said method of treatment expresses CD19, GD2, or CD276 on its surface.

33. The antigen-binding domain for use in the method of treatment according to any one of the embodiments 27-32, wherein the cancer is a hematological cancer or a solid tumor, preferably wherein the solid tumor is breast cancer, colon cancer, lung cancer, skin cancer, brain cancer, head and neck cancer, soft tissue cancer, gastrointestinal cancer, genitourinary and gynecologic cancer, musculoskeletal and bone cancer or endocrine cancer, a breast cancer, a sarcoma or a brain cancer.

34. The antigen-binding domain for use in the method of treatment according to embodiment 33, wherein the hematological cancer is a lymphoma or a leukemia.

35. The antigen-binding domain for use in the method of treatment according to embodiment 34, wherein the lymphoma is Non-Hodgkin Lymphoma, Hodgkin lymphoma, Burkitt Lymphoma, multiple myeloma or follicular lymphoma.

36. The antigen-binding domain for use in the method of treatment according to the embodiment 34, wherein the leukemia is Lymphoblastic Leukemia (ALL), Acute Myeloid Leukemia (AML), Chronic Lymphocytic Leukemia (CLL), Chronic Myeloid Leukemia (CML), Chronic Myelomonocytic Leukemia (CMML), Hairy Cell Leukemia (HCL), Prolymphocytic Leukemia (PLL), Myelodysplastic Syndromes (MDS), or Mixed-Phenotype Acute Leukemia (MPAL).

37. The antigen-binding domain for use in the method of treatment according to the embodiment 33, wherein the breast cancer is triple-negative breast cancer, HER2-positive breast cancer or hormone receptor-positive breast cancer.

38. The antigen-binding domain for use in the method of treatment according to the embodiment 33, wherein the solid tumour is melanoma, osteosarcoma, retinoblastoma, pediatric rhabdomyosarcoma, sarcoma or small cell lung cancer.

39. The antigen-binding domain for use in the method of treatment according to embodiment 33, wherein the brain cancer is a glioblastoma or neuroblastoma.

40. The antigen-binding domain according to any of embodiments 1-26 for use in an immunomodulating therapy.

41. A method of generating an antigen-binding domain

comprising a heavy chain variable region (VH) and, optionally, also a light chain variable region (VL); and wherein the method comprises the steps of:

a) selecting at least one amino acid residue suitable for cysteine substitution, wherein said at least one amino acid residue is comprised within a CDR of VH or VL, has a solvent-accessible surface area from 0.00 to 0.75 and is located at a distance to the antigen from 3 to 14 Å, as defined based on a structural model of an antigen and antigen binding domain
b) mutating selected residue to a thiol-group containing amino acid;
c) testing the binding of the thiol-group containing amino acid engineered antigen-binding domain to the antigen, ideally by surface plasmon resonance (SPR), enzyme-linked immunosorbent assay (ELISA), fluorescence resonance energy transfer (FRET), Isothermal Titration Calorimetry (ITC), or MicroScale Thermophoresis (MST).

42. The method of embodiment 41, wherein at least one amino acid residue suitable for cysteine substitution has a solvent-accessible surface area from 0.00 to 0.70, more preferably from 0.00 to 0.65, even more preferably from 0.00 to 0.60, even more preferably from 0.00 to 0.55, and most preferably from 0.00 to 0.50.

43. The method of the embodiment 41 or 42, wherein at least one amino acid residue suitable for cysteine substitution is located at the distance to the antigen of 3.5-13 Å, even more preferably of 4-12 Å, even more preferably of 4-11 Å, even more preferably of 4-9 Å, and most preferably of 4-8 Å.

**Examples**

The examples provided herein offer experimental evidence for cysteine-engineered antigen-binding domains, such as those incorporated into nanobodies or chimeric antigen receptors (CARs) used in CAR-T cell therapy. These examples are not intended to limit the scope of the invention. A person skilled in the art will recognize that this experimental evidence serves as proof of concept for the broader applicability of cysteine-engineered antigen-binding domains, encompassing, for instance, therapeutic antibodies, nanobodies or bispecific T-cell engagers.

**Example 1.**

**CB2-CAR-T cells specifically target SC-1 lymphoma but not healthy PBMCs**

In the previous study by the inventors, it was demonstrated how CB2 nanobodies specifically target BCL cells via thiol interactions and exploit this mechanism for drug delivery.[8]Therefore, the first objective of this invention was to investigate whether CB2 is also suitable to guide CAR-T cells for thiol-dependent targeting of BCL (Figure 1A). For that, the inventors designed a second-generation CAR construct with CB2 as antigen fragment, human IgG1-Fc as hinge, and CD28 as transmembrane and costimulatory domains. GFP was part of the same vector and served as a reporter protein (CB2-CD28-CAR_GFP; Figure 1B).

Primary human T cells were successfully activated and transduced with lentivirus carrying the CB2-CD28-CAR_GFP or GFP control construct. For the CAR, a relatively low transduction efficiency of approximately 15% was achieved but the CAR-T cells were successfully enriched via FACS (Figure 1C; Figure 9). Immunophenotyping of CAR-T cells revealed that the CD4/CD8 ratio was comparable to non-transduced T cells (Figure 1D).

To test the CAR-T cell activity, co-cultures with SC-1 cells (follicular lymphoma) were performed at different Effector to Target (E:T) ratios and apoptosis of the target cells was measured via flow cytometry. CB2-CAR-T cells exhibited significant concentration-dependent cytotoxicity compared with non-transduced and GFP control T cells (>50% at 9:1 ratio). Furthermore, co-cultures with healthy human PBMCs showed no cytotoxic activity (Figure 2A). To further profile CAR-T cell activation, the inventors performed ELISAs to test the secretion of pro-inflammatory cytokines IFN-$\gamma$ and TNF-$\alpha$ upon co-incubation with SC-1 cells. In agreement with the apoptosis assay results, CB2-CAR-T cells secreted approximately 4-fold higher amounts of both cytokines than control T cells, while healthy PBMCs did not trigger cytokine secretion (Figure 2B). To confirm activity on the effector cell level, overexpression of T cell activation markers, namely CD25 and CD69, and the degranulation marker LAMP 1 was assessed via flow cytometry. All three markers were significantly overexpressed in the case of CB2-CAR-T cells co-incubated with SC-1 (54%, 22%, and 39% for CD25, CD69, and LAMP-1, respectively), confirming T cell activation and degranulation (Figure 2C-D).

These results demonstrate the CB2-CAR-T cell activation upon co-culture with SC-1 lymphoma. More precisely, CAR-T cells exhibit cytotoxicity, secrete pro-inflammatory cytokines, and overexpress T cell activation markers. Based on these findings, the inventors were intrigued to inspect the therapeutic scope of CB2-CAR-T cells against other BCL subtypes and antigen escape models.

**Example 2.**

**CB2-CAR-T cells are effective against BCL subtypes and antigen escape models**

To compare the activity of CB2-CAR-T cells with a standard CD19-CAR-T therapy, the inventors changed the receptor configuration to match the clinically used Kymriah® format (CD19-CAR; Figure 3A), which was used as a positive control. For this, the inventors introduced CD8-derived hinge and transmembrane domains and the 4-1BB-derived costimulatory domain (CB2-41BB-CAR; Figure 3A) and generated CB2-41BB-CAR-T and CD19-CAR-T cells by lentiviral transduction with high efficiencies (>60%; Figure 3B).

To evaluate cytotoxicity, CAR-T cells were co-cultured with luciferase-expressing SC-1 cells, JeKo-1 cells (mantle cell lymphoma), and JeKo-1 cells with knocked-out CD19 or CD20 to model antigen escape (Figure 11). To demonstrate clinical relevance, the inventors pooled data from three independent experiments representing different blood donors for CAR-T cells (N=3, n=3). CB2-CAR-T cells exhibited significant cytotoxicity in a concentration-dependent manner against all tested BCL subtypes (Figure 3C). JeKo-1 CD19-knockout cells were targeted with high activity (≈80% specific lysis at 9:1 ratio), while they were resistant to CD19-CAR-T cells. For other BCL lines, the cytotoxicity of CB2-CAR-T cells was lower compared with CD19-CAR-T cells but consistently reached values above 50% (Figure 3C).

In conclusion, CB2-CAR-T cells are active against different BCL subtypes as well as antigen escape models. Moreover, a BCL line resistant to conventional CD19-directed CAR-T cells can be targeted efficiently. Therefore, the inventors aimed to investigate whether cysteine engineering could be applied to other nanobody-based CAR-T cells.

### Example 3.

**Cysteine-engineered nanobodies can redirect CAR-T cells to target BCL**

As a proof-of-concept, the inventors chose to engineer the non-cancer-related anti-GFP nanobody LaG-16 with a cysteine in CDR3. As described in the previous publication by the inventors, the cysteine-to-serine mutant C105SCB2 exhibits no binding to SC-1 cells in flow cytometry.[8] Therefore, the inventors chose serine 108 in the CDR3 sequence of LaG-16 and introduced a reverse mutation, leading to S108CLaG-16. To assess whether this cysteine-mutant of LaG-16 could bind to SC-1 cells, the inventors performed flow cytometry binding assays. Indeed, S108CLaG-16 showed the same binding pattern as the wild type of CB2, whereas the wild type of LaG-16, like C105SCB2, did not bind (Figure 4A). Based on this result, it was hypothesized that the activity of CB2-CAR-T cells depends exclusively on the exposed cysteine in CDR3, and that nanobody-CARs of different specificity can be redirected towards BCL when endowed with such a cysteine.

To test this hypothesis, the inventors generated CAR-T cells expressing wild type CB2, C105SCB2, wild type LaG-16, and S108CLaG-16 (Figure 8). Transduction efficiencies differed repeatedly between constructs (Figure 4B), but the cell numbers in all assays were adjusted to reach the same amounts of effector cells. The inventors performed luciferase-based cytotoxicity assays with SC-1 cells to compare the activity of the different constructs. In accordance with the cell binding assays, both constructs comprising a cysteine in CDR3 (CB2-CAR and S108CLaG-16-CAR) showed significant cytotoxicity (>50% at 9:1 ratio), while the serine comprising counterparts (C105SCB2-CAR and LaG-16-CAR) did not show any activity. Moreover, activity of S108CLaG-16-CAR-T cells was higher at each E:T ratio compared to CB2-CAR-T cells (Figure 4C).

These results demonstrated how a non-cancer-related nanobody-based CAR-T cells can be redirected to target BCL by a single cysteine substitution on their CDR. Furthermore, replacement of this cysteine by serine leads to a complete loss of the T cells cytotoxic activity.

### Example 4.

**Cysteine-engineering is applicable to scFv-based CD19-CAR and enables targeting of both CD19+ and CD19-lymphoma**

Encouraged by these results, the inventors turned to assess whether cysteine engineering can be applied to the clinically approved CD19-specific FMC63 scFv-based CAR. Thus, the inventors tested several cysteine-substituted CAR constructs. The cysteine mutations were chosen on the heavy (HC) and light (LC) chains of the scFv based on their locations in the CDR sequences of FMC63 and their surface exposure to ensure accessibility of the cysteine residue. In specific, N92- LC-CDR3, D156-HC-CDR1, S190-HC-CDR2, and G228 and S229-HC-CDR3 (Figure 5A-B). The two adjacent residues in HC-CDR3 were chosen to test whether the specific location in the CDR, as well as the identity of the mutated residue, influences activity. CAR-T cells were generated and co-cultured with JeKo-1 lymphoma to assess cytotoxicity. As anticipated, all constructs efficiently targeted CD19-expressing JeKo-1 cells, indicating that none of the mutations significantly interferes with CD19 binding (Figure 5C upper panel).

For the JeKo-1 CD19-knockout, mutations located in HC-CDR1 and HC-CDR2 showed only a slight increase in cytotoxicity compared to the WTCD19-CAR, indicating an impaired suitability of these CDRs for cysteine engineering. However, the mutations in both LC- and HC-CDR3 exhibited significantly higher cytotoxicity compared to WTCD19-CAR (Figure 5C lower panel). Therefore, S229CCD19-CAR was chosen as the candidate mutant for further in vivo inspections.

To confirm optimal cytotoxic activity and demonstrate clinical relevance, co-culture experiments were repeated three times with WTCD19-CAR- and S229CCD19-CAR-T cells generated from three independent blood donors. The significant cytotoxic effect of the S229C mutant against CD19-knockout lymphoma was confirmed (Figure 5D right panel), while CD19-expressing JeKo-1 cells were efficiently targeted by both constructs (Figure 5D left panel).

### Example 5.

**S229CCD19-CAR-T cells prolong the survival of lymphoma-xenografted mice.**

For translation towards in vivo experiments, the inventors examined the effect of S229CCD19-CAR T cells in a mouse antigen escape model. Initially, the safety and off-target damage of the engineered S229CCD19-CAR T cells were examined by injecting them into healthy mice. All mice survived for over 42 days post-treatment. Therefore, the inventors injected xenografted mice with a 1:1 ratio of CD19-positive and CD19-negative lymphoma and subsequently treated those mice with CAR-T cells. Interestingly, in corroboration with the previous in vitro results, mice treated with S229CCD19-CAR-T cells show a delay in tumor formation as well as a prolonged survival compared to WTCD19-CAR-T cells (Figure 7). - As shown in Figure 7A, mice not receiving a CAR (Tumor only) only survived at most 8 days. Mice treated with a WTCD19-CAR-T cells survived at most 15 days post CAR injection. Only mice treated with S229CCD19-CAR-T survived at least 25 days. Figures 7B and 7C depict the luciferase signal over time and it is immediately clear that only the S229CCD19-CAR-T cell treated mice showed less luciferase signal over time, a decrease in tumor growth, and thus also lived significantly longer. These results indicate that thiol-dependent targeting of antigen-escaped lymphoma is effective *in vivo.*

**Example 6.**

**Cysteine-engineering of scFv-based GD2-CAR for targeting of GD2+ and GD2-Triple-Negative Breast Cancer (TNBC) cells**

Encouraged by the CD19 results in mice, the inventors will assess whether cysteine engineering can be applied to the clinically tested GD2-specific 14G2a-scFv-based CAR against Triple-Negative Breast Cancer cells (TNBC). GD2 is a glycolipid recently demonstrated as a valid target in the highly lethal TNBC, but with varying expression levels between patients, from high expression to undetected GD2 levels. Importantly, the authors already demonstrated that abnormal cell membrane redox state on several breast cancer cell lines, including the MDA-MB-231 TNBC cell line.[25] Thus, the inventors aim to test three cysteine-substituted CAR constructs. The cysteine mutations are chosen on the heavy (HC) and light (LC) chains of the scFv based on previous experience from the CD19-CAR construct and available GD2-scFv crystal structures. Their chosen location in the CDR sequences of HC-CDR3 and LC-CDR3 aimed to ensure optimal surface exposure and accessibility of the cysteine residue. Tested mutants are M100C (HC-CDR3; SASA 0.07; distance 4.1 Å), H226C (LC-CDR3; SASA 0.33; distance 6.1 Å), and V227C (LC-CDR3; SASA 0.46; distance 3.6 Å). CAR-T cells will be generated and co-cultured with WT MDA-MB-231 TNBC cells not expressing GD2 to assess their cytotoxicity against antigen-negative target cells. It is expected that all mutant constructs will enable target cell killing, while WT GD2-CAR should be inactive against MDA-MB-231 cells. Next, we will confirm cytotoxicity of all GD2-CAR constructs, including WT GD2-CAR, against the GD2-positive TNBC cell line Hs 578T.Co-culture experiments are planned to be repeated three times with WT GD2-CAR- and mutant GD2-CAR-T cells, generated from three independent blood donors.

**Example 7.**

**Cysteine-engineering of scFv-based CD276-CAR for targeting of CD276+ and CD276- sarcoma cells**

The inventors will additionally assess the suitability of a scFv-based CAR against the tumor-associated antigen CD276 for cysteine engineering. First, the CD276-expressing sarcoma cell line Rh-30 will be investigated for altered redox states on the cell surface, enabling thiol-dependent targeting. Second, a CD276 knockout of Rh-30 will be generated through shRNA silencing. Next, cysteine engineered CD276-CAR-T cells will be generated and tested in cytotoxicity assays against CD276-positive and -negative Rh-30 cells as described above

**Materials and Methods**

Design of CAR constructs

The second-generation CAR construct comprising human elongation factor-1 alpha promotor (EF-1α) → CD8 signal peptide (SP) (SEQ ID NO: 25) → CB2 domain (SEQ ID NO: 17) → human IgG1-Fc hinge (SEQ ID NO: 20) → CD28 transmembrane domain (TM) (SEQ ID NO: 22) → CD28 costimulatory domain (CD) (SEQ ID NO: 23) → CD3ζ activating domain (AD) (SEQ ID NO: 25) → Woodchuck Hepatitis Virus Posttranscriptional Regulatory Element (WPRE) → human cytomegalovirus enhancer and promotor (CMV) → green fluorescent protein (GFP), abbreviated "CB2-CD28-CAR_GFP", was ordered as synthetic gene in the lentiviral transfer plasmid pLV from VectorBuilder Inc. (Chicago, IL). GFP control construct ("GFP") was cloned in-lab via restriction of the plasmid with SpeI followed by self-ligation.
A shorter version of the construct, abbreviated "CB2-CD28-CAR", was generated via deletion of GFP reporter using primers SEQ ID NO: 52 and 54. Briefly, the complete CAR construct was amplified via polymerase chain reaction (PCR) using a reverse primer with an overhang to insert a 3' KpnI restriction site. Then, the amplicon and the original backbone were restricted using XbaI (restriction site upstream CB2 domain) and KpnI (restriction site downstream GFP) and ligated. The [C105S]CB2 mutant of this construct was generated via overlap extension PCR as described by Heckman et al.[32], leading to "[C105S]CB2-CD28-CAR". Sequences of the anti-GFP nanobody LaG-16 and its mutant [S108C]LaG-16 were ordered as synthetic gene fragments from IDT Inc. (Coralville, IA). The sequence was published by Fridy et al.[33] The gene fragments were inserted into the CD28-CAR backbone via NEBuilder® HiFi DNA Assembly (NEB, Ipswich, MA), leading to "LaG-16-CD28-CAR" and "[S108C]LaG-16-CD28-CAR".

Furthermore, a second-generation CAR construct comprising granulocyte-macrophage colony-stimulating factor (GM-CSF) SP (SEQ ID NO: 27) instead of CD8-SP, CD8 hinge (SEQ ID NO: 19) instead of IgG1-Fc hinge, CD8-TM (SEQ ID NO: 21) instead of CD28-TM, and 4-1BB-CD (SEQ ID NO:24) instead of CD28-CD was generated. The original plasmid was provided by the Seitz lab, university of Tübingen, with anti-human CD19 single-chain variable fragment FMC63 as extracellular antibody fragment (abbreviated "CD19-CAR"). To clone the construct "CB2-41BB-CAR", FMC63 was replaced by CB2 via NEBuilder® HiFi DNA Assembly.

The FMC63-CAR cysteine mutants N92C, D156C, S190C, G228C, and S229C were ordered as codon-optimized synthetic DNA fragments (IDT, Coralville, IA, USA) and inserted into the pLV:CD19-CAR backbone via restriction and ligation cloning using EcoRV and NdeI (NEB, Ipswich, MA, USA) according to the manufacturer's instructions.

Lentiviral constructs used in this study are summarized in Figure 8.

Lentiviral packaging

HEK293T cells were transfected with pLV (carrying the respective CAR construct), psPAX2 (plasmid was a gift from Didier Trono, Addgene plasmid # 12260; http://n2t.net/addgene:12260; RRID:Addgene_12260), and pMD2.G (plasmid was a gift from Didier Trono, Addgene plasmid # 12259; http://n2t.net/addgene:12259; RRID:Addgene_12259) in a T75 flask with 6.3 μg DNA per plasmid and 30 μl TransIT 293T transfection reagent (Mirus Bio LLC, Madison, WI). Cells were incubated at 37°C for 48 h. Afterwards, viral supernatant was centrifuged at 1500 g for 10 min and filtered through a 0.45 μm filter. For concentration, supernatant was layered on a sucrose buffer (50 mM Tris-HCl, 100 mM NaCl, 0.5 mM EDTA, 10% w/v sucrose, pH 7.4) at 4:1 v/v ratio in a 50 ml tube and centrifuged at 10 000 g/4°C for 4 h. Afterwards, the supernatant was removed, and the viral pellet was resuspended in 500 μl sterile PBS. For storage, virus suspensions were aliquoted and frozen directly at -80°C. Lentivirus concentration protocol was adapted from Jiang et al.[34]

CAR-T cell generation

Human whole blood samples were obtained from the German Red Cross (DRK-Blutspendedienst Nord-Ost, Institut Berlin). PBMCs were isolated using SepMate™-50 tubes (Stemcell Technologies, Vancouver, Canada). Afterwards, T cells were isolated using Pan T Cell Isolation Kit (Miltenyi Biotec, Bergisch Gladbach, Germany) and seeded at 1.0 million cells/ml in T cell medium (RPMI 1640, 10% fetal calf serum (FCS), 2 mM L-glutamine, 1 mM pyruvate, 100 U/ml Penicillin-Streptomycin) supplemented with 30 U/ml human Interleukin-2 (IL-2; Peprotech Inc., Cranbury, NJ). 25 μl Dynabeads™ Human T-Activator CD3/CD28 beads (ThermoFisher Scientific, Waltham, MA) per million of T cells were added. One day later, concentrated lentivirus was added in a 1:15 dilution alongside with 5 μg/ml polybrene. On day 2, medium was replaced with fresh T cell medium + IL-2. Cells were monitored daily and maintained between 0.5 and 1.0 million cells/ml. T cells transduced with CB2-CD28-CAR_GFP and GFP control were enriched via fluorescence-activated cell sorting (see respective section) on day 6. All other constructs were not sorted but transduction efficiencies were determined via flow cytometry. Briefly, all nanobody-based CAR-T cells were incubated with MonoRab™ Rabbit Anti-Camelid VHH Cocktail iFluor 647 (1:500; GenScript, Piscataway, NJ) and CD19-CAR-T cells were incubated with Recombinant Human CD19 Fc Chimera Alexa Fluor® 647 (1:500; Bio-Techne, Minneapolis, MN) at room temperature (RT) for 30 min. Samples were measured on a FACSCanto™ II Flow Cytometry System (BD, Franklin Lakes, NJ). 24 h before experiments, T cells were transferred into medium without IL-2. For long-term storage, CAR-T cells were cryopreserved in liquid nitrogen with 90% FCS, 10% dimethyl sulfoxide as freezing medium.

Fluorescence-activated cell sorting (FACS)

T cells transduced with CB2-CD28-CAR_GFP were stained with MonoRab™ Rabbit Anti-Camelid VHH Cocktail iFluor 647 at 4°C for 30 min. GFP control T cells were resuspended in PBS. Subsequently, cells were sorted for nanobody-positive or GFP-positive cells respectively, using a FACSMelody™ Cell Sorter (BD, Franklin Lakes, NJ). Sorting efficiency was determined afterwards using the same staining pattern (Figure 9).

Immunophenotyping of CAR-T cells (CD4/CD8 ratio)

The CD4/CD8 ratio of non-transduced and CB2-CAR-T cells was determined via flow cytometry. T cells were stained with MonoRab™ Rabbit Anti-Camelid VHH Cocktail iFluor 647, PE anti-human CD4 Antibody (1:100; BioLegend, San Diego, CA), and FITC anti-human CD8 Antibody (1:100; BioLegend, San Diego, CA) at RT for 30 min. Each condition was prepared in triplicates. Samples were measured on a FACSCanto™ II and events were gated for single cells and, in the case of CB2-CAR-T cells, also for CAR-positive cells.

Apoptosis assays

Non-transduced, sorted GFP control and sorted CB2-CAR-T cells were co-cultivated with SC-1 cells in different Effector to Target (E:T) ratios (1:1, 3:1, 5:1 and 9:1). Each condition was prepared in triplicates. SC-1 cell numbers were constant ($5 \times 10^3$ cells/well). Co-cultivation took place in a 96-well plate for 72 h at 37°C. For analysis, cells were stained with anti-CD3-APC antibody (1:200; BioLegend, San Diego, CA) to distinguish B cell lymphoma (BCL) and T cells, Annexin V-PE (1:100; BioLegend, San Diego, CA) to stain early apoptotic cells and 7-AAD Viability Staining Solution (1:100; ThermoFisher Scientific, Waltham, MA) to stain late apoptotic cells. Incubation took place in Annexin V Binding Buffer (BioLegend, San Diego, CA) at RT for 15 min. Cells from each well were analyzed separately on a FACSCanto™ II. Target cells were gated for single cells and SC-1 cells (CD3-/GFP- events; Figure 10). Average frequencies of early and late apoptotic SC-1 cells were compared between the different conditions and E:T ratios.

Apoptosis assays with healthy human PBMCs were performed as described above, but PBMCs were stained with CellTrace™ Far Red Cell Proliferation Kit (ThermoFisher Scientific, Waltham, MA) prior to co-culture to distinguish them from CAR-T cells and replace the use of anti-CD3 antibody. Therefore, healthy PBMCs were gated for CellTrace™+/GFP- events.

Significance of differences was assessed via 2-way ANOVA followed by Tukey's post-hoc test.

Cytokine secretion assays

Cytokine levels were measured via enzyme-linked immunosorbent assay (ELISA). Supernatants (9:1 ratio) of the co-culture assays described above were collected and levels of IFN-$\gamma$ and TNF-o were determined in triplicates using Human IFN-$\gamma$ and Human TNF-o Mini TMB ELISA Kits (Peprotech Inc., Cranbury, NJ). Samples of T cells only were included to determine baseline cytokine secretion. Significance of differences was assessed via 2-way ANOVA followed by Tukey's post-hoc test.

Analysis of T cell activation marker expression

T cells were co-cultivated with SC-1 cells in an E:T ratio of 9:1 in triplicates as described above. Subsequently, each replicate was split into three equal fractions and stained with one of the antibodies for marker detection (PE/Cyanine7 anti-human CD25, PE/Cyanine7 anti-human CD107a (LAMP-1) or PE/Cyanine7 anti-human CD69; all BioLegend, San Diego, CA). Anti-CD3-APC antibody (BioLegend, San Diego, CA) was included in all samples to gate for T cells. Baselines of marker expression were determined with T cells only samples. Marker expression was measured on a FACSCanto™ II. Expression levels of T cells co-cultivated with SC-1 cells were subtracted with expression levels of T cells only. Significance of differences for each marker was assessed via 1-way ANOVA followed by Tukey's post-hoc test.

Cell culture

SC-1 and HEK293T cell lines were purchased from DSMZ, Braunschweig, Germany. JeKo-1 and its knockouts were kindly provided by the Seitz lab, university of Tübingen. All cell lines were maintained in a humidified incubator at 37°C and 5% $CO_2$. HEK293T cells were cultured in DMEM medium supplemented with 10% FCS, 2 mM L-glutamine, and 100 U/ml Penicillin-Streptomycin. All BCL lines were cultured in RPMI 1640 medium supplemented with 10% FCS, 2 mM L-glutamine, 1 mM pyruvate and 100 U/ml Penicillin-Streptomycin. For culture conditions of CAR-T cells, see respective section. All cell culture media and supplements were purchased from PAN-Biotech GmbH, Aidenbach, Germany. Cell cultures were tested negative for mycoplasma via PCR monthly.

JeKo-1 lymphoma knockout cell lines

JeKo-1 cells and the knockout lines JeKo-1 CD19-KO and JeKo-1 CD20-KO, all expressing firefly luciferase, were generated by the Seitz lab, university of Tübingen, as described elsewhere.[20] Knockout efficiencies were determined via flow cytometry (Figure 11) incubating with PE anti-human CD19 Antibody (1:100) and APC anti-human CD20 Antibody (1:100; both BioLegend, San Diego, CA) at RT for 1 h.

Generation of luciferase-expressing SC-1 cells

SC-1 cells expressing firefly luciferase and GFP were generated in-lab via lentiviral transduction. Lentiviral particles were produced as described above with SIN40C.SFFV.Luciferase.IRES.GFP as transfer plasmid (plasmid was a gift from Jan-Henning Klusmann, Addgene plasmid # 169308; http://n2t.net/addgene:169308; RRID:Addgene_169308).[35] One million SC-1 cells were transduced with 120 μl concentrated lentivirus and 8 μg/ml polybrene. On day 8, cells were single-cell sorted for GFP-positive cells using a FACSMelody™. Expanding clones were screened on day 23 for uniform GFP expression. One clone was selected, further expanded and cryo-preserved. The SC-1 origin was confirmed via flow cytometry with PE anti-human CD19 Antibody (1:100; BioLegend, San Diego, CA), confirming uniform CD19 and GFP expression of the clone (Figure 12).

Luciferase-based cytotoxicity assays

Non-transduced and CAR-T cells were co-cultivated with BCL in different E:T ratios (1:1, 3:1, 5:1 and 9:1). Each condition was prepared in triplicates. BCL cell numbers were constant ($5 \times 10^3$ cells/well). BCL only samples were included for lysis control. Differences in CAR-T cell transduction efficiencies were considered by adjusting T cell numbers to reach the same amounts of CAR-positive cells among all constructs. Co-cultivation took place in a 96-well plate at 37°C for 24 h (SC-1) or 48 h (all other BCL). For analysis, 100 μg/ml D-luciferin potassium salt (Abcam, Cambridge, UK) was added to each well. For lysis control wells, 1% NP-40 was added. Cells were incubated at 37°C for 1 h. Afterwards, relative light units (RLU) were measured in a CLARIOstar Plus plate reader (BMG LABTECH GmbH, Ortenberg, Germany) with an exposure time of 10 seconds per well. Normalized specific lysis (Normalized spec. lysis) was calculated by normalizing test RLUs to target cells co-cultured with non-transduced T cells (NT) at the respective E:T ratio (background cytotoxicity) and lysis control wells (maximal killing) using the following equation:

$$\text{Normalized spec. lysis } [\%] = 100 \frac{RLU_{NT} - RLU_{test}}{RLU_{NT} - RLU_{lysis\ control}}$$

If indicated in the figure legend, data from independent experiments was pooled by calculating the grand mean (mean of means) and pooled standard error (SEM$_p$) via the following equation:

$$SEM_p = \sqrt{\frac{\sum_{i=1}^{k}(n_i - 1)s_i^2}{\sum_{i=1}^{k}(n_i - 1)} \sum_{i=1}^{k} \frac{1}{n_i}}$$

Where $n_i$ is the sample size of the i-th independent experiment; $s_i^2$ is the variance of the i-th independent experiment; and k is the number of independent experiments. Significance of differences was assessed via 2-way ANOVA followed by Tukey's post-hoc test.

Recombinant expression of nanobodies in *Escherichia coli*

Histidine-tagged LaG-16 expression plasmid "pET21-pelB-LaG-16" was a gift from Michael Rout (Addgene plasmid #172746; http://n2t.net/addgene:172746; RRID:Addgene_172746).[33] The S108C mutation was introduced by overlap-extension PCR as described elsewhere.[32] Both plasmids were transformed into ArcticExpress (DE3) *Escherichia coli* cells (Agilent Technologies, Santa Clara, CA), and protein expression was induced in lysogenic broth medium with 1 mM isopropyl-β-D-1-thiogalactopyranoside at 12°C overnight. Bacteria were lysed with 5 freeze-thaw cycles, alternating between a 37°C water bath and dry ice. Nanobody was then purified in two steps, first by Nickel-NTA agarose resin (ThermoFisher Scientific, Waltham, MA) followed by size exclusion chromatography on an ÄKTApurifier using an S200 column (Cytiva, Marlborough, MA). All steps and purity of the final product were validated by sodium dodecyl sulfate polyacrylamide gel electrophoresis (SDS-PAGE, Figure 13). CB2 and [C105S]CB2 were expressed and purified as described elsewhere.[25]

Flow cytometry nanobody binding assays

One million SC-1 cells per sample were incubated with 24 μM nanobody solution or PBS (negative control) at RT for 1 h. Afterwards, all samples were incubated with MonoRab™ Rabbit Anti-Camelid VHH Cocktail iFluor 647 at RT for 1 h. Samples were measured on a FACSCanto™ II.

*In vivo* experiments

Immunodeficient mice (n=3 per group) were xenografted with human lymphoma cell lines JeKo-1 and JeKo-1 CD19-KO in a 1:1 mixture at $0.5 \times 10^6$ cells each per mouse on day -6. Both cell lines are expressing GFP and firefly luciferase. On day 0, CAR-T cells were injected. Groups tested were tumor only, mock T cells, CD19-CAR, and [S229C]CD19-CAR. Tumor burden was measured via the luciferase signal on days -1, 1, 4, 6, 8, 10, 13, 15, 17, 21, 23, and 25.

**Table of Sequences**

| SEQ ID NO | Type | Description | Sequence |
|---|---|---|---|
| 1 | AA | anti-CD19 scFv FMC63 | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGSYAMDYWGQGTSVTVSS |
| 2 | AA | anti-CD19 scFv FMC63 S229C | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGCYAMDYWGQGTSVTVSS |
| 3 | AA | anti-CD19 scFv FMC63 G228C | DIQMTQTTSSLSASLGDRVTISCRASQDISKYLNWYQQKPDGTV KLLIYHTSRLHSGVPSRFSGSGSGTDYSLTISNLEQEDIATYFCQQ GNTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGCSYAMDYWGQGTSVTVSS |
| 4 | AA | anti-CD19 scFv FMC63 N92C | DIQMTQTTSSLSASLGDRVTISCRASQDISKYLNWYQQKPDGTV KLLIYHTSRLHSGVPSRFSGSGSGTDYSLTISNLEQEDIATYFCQQ GCTLPYTFGGGTKLEITGSTSGSGKPGSGEGSTKGEVKLQESGPG LVAPSQSLSVTCTVSGVSLPDYGVSWIRQPPRKGLEWLGVIWGS ETTYYNSALKSRLTIIKDNSKSQVFLKMNSLQTDDTAIYYCAKHY YYGGSYAMDYWGQGTSVTVSS |
| 5 | AA | CB2 | GHVQLVESGGGLVQPGGSLRLSCAASGFTFSNYRMYWIRQAPGK GLEWVSTISSDGVATYYADSVKGRFTISRDNAKNTLYLQMNRLK LEDTAVYSCAALNRYCGNEYEYDYRGQGTQVTVSS |
| 6 | AA | LAG16 WT | QVQLVESGGRLVQAGDSLRLSCAASGRTFSTSAMAWFRQAPGR EREFVAAITWTVGNTILGDSVKGRFTISRDRAKNTVDLQMDNLE PEDTAVYYCSARSRGYVLSVLRSVDSYDYWGQGTQVTVSS |
| 7 | AA | CD8 hinge | TTTPAPRPPTPAPTIASQPLSLRPEACRPAAGGAVHTRGLDFACD |

(continued)

| SEQ ID NO | Type | Description | Sequence |
|-----------|------|-------------|----------|
| 8 | AA | IgG Fc hinge | EPKSCDKTHTCPPCPAPELLGGPSVFLFPPKPKDTLMISRTPEVTC VVVDVSHEDPEVKFNWYVDGVEVHNAKTKPREEQYNSTYRVVS VLTVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPVD |
| 9 | AA | CD8 TM | IYIWAPLAGTCGVLLLSLVITLYC |
| 10 | AA | CD28 TM | FWVLVVVGGVLACYSLLVTVAFIIFWV |
| 11 | AA | CD28 CD | GSRSKRSRLLHSDYMNMTPRRPGPTRKHYQPYAPPRDFAAYRS |
| 12 | AA | 4-1BB CD | KRGRKKLLYIFKQPFMRPVQTTQEEDGCSCRFPEEEEGGCEL |
| 13 | AA | CD3 ζ AD | RVKFSRSADAPAYQQGQNQLYNELNLGRREEYDVLDKRRGRDP EMGGKPRRKNPQEGLYNELQKDKMAEAYSEIGMKGERRRGKGH DGLYQGLSTATKDTYDALHMQALPPR |
| 14 | AA | CD8 SP | MALPVTALLLPLALLLHAARP |
| 15 | AA | GM-CSF | MLLLVTSLLLCELPHPAFLLIP |
| 16 | DNA | anti-CD19 scFv FMC63 | gacatccagatgacccagaccacaagcagcctgtctgccagcctgggcgataga gtgaccatcagctgtagagccagccaggacatcagcaagtacctgaactggtatc agcaaaagcccgacggcaccgtgaagctgctgatctaccacaccagcagactgc acagcggcgtgccaagcagattttctggcagcggctctggcaccgactacagcct gacaatcagcaacctggaacaagaggatatcgctacctacttctgccagcaaggc aacaccctgccttacacctttggcggaggcaccaagctggaaatcaccggctctac aagcggcagcggcaaacctggatctggcgagggatctaccaagggcgaagtga aactgcaagagtctggccctggactggtggccccatctcagtctctgagcgtgacc tgtacagtcagcggagtgtccctgcctgattacggcgtgtcctggatcagacagcct cctcggaaaggcctggaatggctgggagtgatctggggcagcgagacaacctac tacaacagcgccctgaagtcccggctgaccatcatcaaggacaactccaagagcc aggtgttcctgaagatgaacagcctgcagaccgacgacaccgccatctactattgc gccaagcactactactacggcggcagctacgccatggattattggggccagggca ccagcgtgacagtttcttca |

(continued)

| SEQ ID NO | Type | Description | Sequence |
|---|---|---|---|
| 17 | DNA | CB2 | GGCCACGTGCAGCTGGTGGAGAGCGGCGGCGGCCTGGTGCAG CCCGGCGGCAGCCTGCGCCTGAGCTGCGCCGCCAGCGGCTTC ACCTTCAGCAACTACCGCATGTACTGGATACGCCAGGCCCCCG GCAAGGGCCTGGAGTGGGTGAGCACCATCAGCAGCGACGGCG TGGCCACCTACTACGCCGACAGCGTGAAGGGCCGCTTCACCAT CAGCCGCGACAACGCCAAGAACACCCTGTACCTGCAGATGAAC CGCCTGAAGCTGGAGGACACCGCCGTGTACAGCTGCGCCGCCC TGAACCGCTACTGCGGCAACGAGTACGAGTACGACTACCGCGG CCAGGGCACCCAGGTGACCGTGAGCAGC |
| 18 | DNA | LAG16 | CAAGTTCAACTGGTGGAAAGTGGCGGGAGACTTGTGCAGGCC GGCGATAGCCTGCGGCTCTCATGTGCAGCCTCTGGCAGAACTT TCTCCACGTCAGCCATGGCCTGGTTTAGACAAGCTCCAGGAAG GGAGCGGGAATTTGTGGCCGCGATTACCTGGACTGTCGGGAA CACTATCCTGGGAGATTCAGTTAAGGGCCGCTTTACCATATCC CGGGATCGGGCTAAGAATACCGTGGACCTCCAGATGGACAACC TTGAGCCTGAAGACACCGCAGTCTACTACTGTGCTGTCCGTGC TGCGGAGCCGCGGCTACGTACTTTGCGTGCTGCGCTCTGTGGA CAGCTACGATTACTGGGGACAGGGCACACAAGTGACAGTCAGC TCT |
| 19 | DNA | CD8 hinge | acaacaacaccagctcctcggcctccaactcctgctcctacaattgcctctcagcctc tgtctctgaggcccgaagcttgtagacctgctgctggcggagctgtgcacaccaga ggactggatttcgcctgcgac |
| 20 | DNA | IgG Fc hinge | GAGCCCAAATCTTGTGACAAAACTCACACATGCCCACCGTGCC CAGCACCTGAACTCCTGGGGGGACCGTCAGTCTTCCTCTTCCC CCCAAAACCCAAGGACACCCTCATGATCTCCCGGACCCCTGAG GTCACATGCGTGGTGGTGGACGTGAGCCACGAAGACCCTGAG GTCAAGTTCAACTGGTACGTGGACGGCGTGGAGGTGCATAATG CCAAGACAAAGCCGCGGGAGGAGCAGTACAACAGCACGTACCG TGTGGTCAGCGTCCTCACCGTCCTGCACCAGGACTGGCTGAAT GGCAAGGAGTACAAGTGCAAGGTCTCCAACAAAGCCCTCCCAG CCCCCATCGAGAAAACCATCTCCAAAGCCAAAGGGCAGCCCCG AGAACCACAGGTGTACACCCTGCCCCCATCCCGGGATGAGCTG ACCAAGAACCAGGTCAGCCTGACCTGCCTGGTCAAAGGCTTCT ATCCCAGCGACATCGCCGTGGAGTGGGAGAGCAATGGGCAGC CGGAGAACAACTACAAGACCACGCCTCCCGTGCTGGACTCCGA |

(continued)

| SEQ ID NO | Type | Description | Sequence |
|---|---|---|---|
| | | | CGGCTCCTTCTTCCTCTACAGCAAGCTCACCGTGGACAAGAGC AGGTGGCAGCAGGGGAACGTCTTCTCATGCTCCGTGATGCATG AGGCTCTGCACAACCACTACACACAGAAGAGCCTCTCCCTGTC TCCGGTCGAC |
| 21 | DNA | CD8 TM | atctacatttgggcccctctggctggaacatgcggagttctgctgctgagcctggtc atcaccctgtactgc |
| 22 | DNA | CD28 TM | TTTTGGGTGCTGGTGGTGGTTGGTGGAGTCCTGGCTTGCTAT AGCTTGCTAGTAACAGTGGCCTTTATTATTTTCTGGGTG |
| 23 | DNA | CD28 CD | GGATCCAGGAGTAAGAGGAGCAGGCTCCTGCACAGTGACTACA TGAACATGACTCCCCGCCGCCCCGGGCCCACCCGCAAGCATTA CCAGCCCTATGCCCCACCACGCGACTTCGCAGCCTATCGCTCC |
| 24 | DNA | 4-1BB CD | aagcggggcagaaagaagctgctgtacatcttcaagcagcccttcatgcggcccg tgcagaccacacaagaggaagatggctgctcctgcagattccccgaggaagaag aaggcggctgcgagctg |
| 25 | DNA | CD3 ζ AD | agagtgaagttctccagatctgccgacgctcctgcctaccagcagggccagaatc agctgtacaacgagctgaacctggggagaagagaagagtacgacgtgctggat aagcggagaggcagagatcctgagatgggcggcaagcccagacggaagaatc ctcaagagggcctgtataatgagctgcagaaagacaagatggccgaggcctaca gcgagatcggaatgaagggcgagcgcagaagaggcaagggccacgatggact gtatcagggcctgagcacagccaccaaggatacctatgatgccctgcacatgcag GCCCTGCCTCCAAGA |
| 26 | DNA | CD8 SP | atggccttaccagtgaccgccttgctcctgccgctggccttgctgctccacgccgcc aggccg |
| 27 | DNA | GM-CSF | atgctgctgctggtcacatctctgctgctgtgcgagctgccccatcctgcctttctgct gatcccc |

(continued)

| SEQ ID NO | Type | Description | Sequence |
|---|---|---|---|
| 28 | RNA | anti-CD19 scFv FMC63 WT | gacatccagatgacccagaccacaagcagcctgtctgccagcctgggcgatagagtgaccatcagctgtagagccagccaggacatcagcaagtacctgaactggtatcagcaaaagcccgacggcaccgtgaagctgctgatctaccacaccagcagactgcacagcggcgtgccaagcagattttctggcagcggctctggcaccgactacagcctgacaatcagcaacctggaacaagaggatatcgctacctacttctgccagcaaggcaacaccctgccttacacctttggcggaggcaccaagctggaaatcaccggctctacaagcggcagcggcaaacctggatctggcgagggatctaccaagggcgaagtgaaactgcaagagtctggccctggactggtggccccatctcagtctctgagcgtgacctgtacagtcagcggagtgtccctgcctgattacggcgtgtcctggatcagacagcctcctcggaaaggcctggaatggctgggagtgatctggggcagcgagacaacctactacaacagcgccctgaagtcccggctgaccatcatcaaggacaactccaagagccaggtgttcctgaagatgaacagcctgcagaccgacgacaccgccatctactattgcgccaagcactactactacggcggcagctacgccatggattattggggccagggcaccagcgtgacagtttcttca |
| 29 | RNA | CB2 | GGCCACGTGCAGCTGGTGGAGAGCGGCGGCGGCCTGGTGCAGCCCGGCGGCAGCCTGCGCCTGAGCTGCGCCGCCAGCGGCTTCACCTTCAGCAACTACCGCATGTACTGGATACGCCAGGCCCCCGGCAAGGGCCTGGAGTGGGTGAGCACCATCAGCAGCGACGGCGTGGCCACCTACTACGCCGACAGCGTGAAGGGCCGCTTCACCATCAGCCGCGACAACGCCAAGAACACCCTGTACCTGCAGATGAACCGCCTGAAGCTGGAGGACACCGCCGTGTACAGCTGCGCCGCCCTGAACCGCTACTGCGGCAACGAGTACGAGTACGACTACCGCGGCCAGGGCACCCAGGTGACCGTGAGCAGC |
| 30 | RNA | LAG16 WT | CAAGTTCAACTGGTGGAAAGTGGCGGGAGACTTGTGCAGGCCGGCGATAGCCTGCGGCTCTCATGTGCAGCCTCTGGCAGAACTTTCTCCACGTCAGCCATGGCCTGGTTTAGACAAGCTCCAGGAAGGGAGCGGGAATTTGTGGCCGCGATTACCTGGACTGTCGGGAACACTATCCTGGGAGATTCAGTTAAGGGCCGCTTTACCATATCCGGGGATCGGGCTAAGAATACCGTGGACCTCCAGATGGACAACCTTGAGCCTGAAGACACCGCAGTCTACTACTGTTCTGCTCGGAGCCGCGGCTACGTACTTAGTGTGCTGCGCTCTGTGGACAGCTACGATTACTGGGGACAGGGCACACAAGTGACAGTCAGCTCT |
| 31 | RNA | CD8 hinge | ACAACAACACCAGCTCCTCGGCCTCCAACTCCTGCTCCTACAATTGCCTCTCAGCCTCTGTCTCTGAGGCCCGAAGCTTGTAGACCTGCTGCTGGCGGAGCTGTGCACACCAGAGGACTGGATTTCGCCTGCGAC |

(continued)

| SEQ ID NO | Type | Description | Sequence |
|---|---|---|---|
| 32 | RNA | IgG Fc hinge | GAGCCCAAATCTTGTGACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATCCCGGGATGAGCTGACCAAGAACCAGGTCAGCCTGACCTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACACAGAAGAGCCTCTCCCTGTCTCCGGTCGAC |
| 33 | RNA | CD8 TM | ATCTACATTTGGGCCCCTCTGGCTGGAACATGCGGAGTTCTGCTGCTGAGCCTGGTCATCACCCTGTACTGC |
| 34 | RNA | CD28 TM | TTTTGGGTGCTGGTGGTGGTTGGTGGAGTCCTGGCTTGCTATAGCTTGCTAGTAACAGTGGCCTTTATTATTTTCTGGGTG |
| 35 | RNA | CD28 CD | GGATCCAGGAGTAAGAGGAGCAGGCTCCTGCACAGTGACTACATGAACATGACTCCCCGCCGCCCCGGGCCCACCCGCAAGCATTACCAGCCCTATGCCCCACCACGCGACTTCGCAGCCTATCGCTCC |
| 36 | RNA | 4-1BB CD | AAGCGGGGCAGAAAGAAGCTGCTGTACATCTTCAAGCAGCCCTTCATGCGGCCCGTGCAGACCACACAAGAGGAAGATGGCTGCTCCTGCAGATTCCCCGAGGAAGAAGAAGGCGGCTGCGAGCTG |
| 37 | RNA | CD3 ζ AD | AGAGTGAAGTTCTCCAGATCTGCCGACGCTCCTGCCTACCAGCAGGGCCAGAATCAGCTGTACAACGAGCTGAACCTGGGGGAGAAGAGAAGAGTACGACGTGCTGGATAAGCGGAGAGGCAGAGATCCTGAGATGGGCGGCAAGCCCAGACGGAAGAATCCTCAAGAGGGCCTGTATAATGAGCTGCAGAAAGACAAGATGGCCGAGGCCTACAGCGAGATCGGAATGAAGGGCGAGCGCAGAAGAGGCAAGGGCCACGATGGACTGTATCAGGGCCTGAGCACAGCCACCAAGGATACCTATGATGCCCTGCACATGCAGGCCCTGCCTCCAAGA |
| 38 | RNA | CD8 SP | ATGGCCTTACCAGTGACCGCCTTGCTCCTGCCGCTGGCCTTGCTGCTCCACGCCGCCAGGCCG |

(continued)

| SEQ ID NO | Type | Description | Sequence |
|---|---|---|---|
| 39 | RNA | GM-CSF SP | ATGCTGCTGCTGGTCACATCTCTGCTGCTGTGCGAGCTGCCCCATCCTGCCTTTCTGCTGATCCCC |
| 40 | AA | anti-GD2 scFv 14G2a | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGMEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTHVPPLTFGAGTKLELKR |
| 41 | AA | anti-GD2 scFv 14G2a M100C | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGCEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTHVPPLTFGAGTKLELKR |
| 42 | AA | anti-GD2 scFv 14G2a H226C | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGMEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTCVPPLTFGAGTKLELKR |
| 43 | AA | anti-GD2 scFv 14G2a V227C | EVQLLQSGPELEKPGASVMISCKASGSSFTGYNMNWVRQNIGKSLEWIGAIDPYYGGTSYNQKFKGRATLTVDKSSSTAYMHLKSLTSEDSAVYYCVSGMEYWGQGTSVTVSSGGGGSGGGGSGGGGSEIVMTQSPATLSVSPGERATLSCRSSQSLVHRNGNTYLHWYLQKPGQSPKLLIHKVSNRFSGVPDRFSGSGSGTDFTLKISRVEAEDLGVYFCSQSTHCPPLTFGAGTKLELKR |
| 44 | DNA | anti-GD2 scFv 14G2a | GAAGTGCAGCTGCTGCAGTCTGGCCCTGAACTGGAAAAACCTGGCGCCTCCGTGATGATCAGCTGTAAAGCCAGCGGCAGCAGCTTCACCGGCTACAACATGAACTGGGTCCGACAGAACATCGGCAAGAGCCTGGAATGGATCGGCGCCATCGATCCTTACTACGGCGGCA |

50

(continued)

| SEQ ID NO | Type | Description | Sequence |
|---|---|---|---|
| | | | CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |
| 45 | DNA | anti-GD2 scFv 14G2a M100C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCTGTG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |

(continued)

| SEQ ID NO | Type | Description | Sequence |
|---|---|---|---|
| 46 | DNA | anti-GD2 scFv 14G2a H226C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACATGTGTGCCACC TCTGACCTTTGGCGCTGGCACAAAGCTGGAACTGAAGCGG |
| 47 | DNA | anti-GD2 scFv 14G2a V227C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACTGTCCACC TCTGACCTTTGGCGCTGGCACAAAGCTGGAACTGAAGCGG |

(continued)

| SEQ ID NO | Type | Description | Sequence |
|---|---|---|---|
| 48 | RNA | anti-GD2 scFv 14G2a | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |
| 49 | RNA | anti-GD2 scFv 14G2a M100C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCTGTG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACACACGTGCCACC TCTGACCTTTGGCGCTGGCACAAAGCTGGAACTGAAGCGG |

| SEQ ID NO | Type | Description | Sequence |
|---|---|---|---|
| 50 | RNA | anti-GD2 scFv 14G2a H226C | GAAGTGCAGCTGCTGCAGTCTGGCCCTGAACTGGAAAAACCTG GCGCCTCCGTGATGATCAGCTGTAAAGCCAGCGGCAGCAGCTT CACCGGCTACAACATGAACTGGGTCCGACAGAACATCGGCAAG AGCCTGGAATGGATCGGCGCCATCGATCCTTACTACGGCGGCA CCAGCTACAACCAGAAGTTCAAGGGCAGAGCCACACTGACCGT GGACAAGAGCAGCAGCACAGCCTACATGCACCTGAAGTCCCTG ACAAGCGAGGACAGCGCCGTGTACTACTGTGTGTCCGGCATGG AATATTGGGGCCAGGGCACAAGCGTGACAGTTTCTTCTGGTGG CGGAGGATCTGGCGGAGGTGGAAGCGGCGGAGGCGGATCTGA AATCGTGATGACACAGAGCCCCGCCACTCTGAGTGTGTCTCCA GGCGAAAGAGCTACCCTGAGCTGTAGAAGCAGCCAGAGCCTG GTGCACAGAAACGGCAATACCTACCTGCACTGGTATCTGCAGA AGCCCGGCCAGTCTCCTAAGCTGCTGATCCACAAGGTGTCCAA CAGATTCAGCGGCGTGCCCGATAGATTTTCTGGCTCTGGCAGC GGCACCGACTTCACCCTGAAGATTAGCAGAGTGGAAGCCGAGG ACCTGGGCGTGTACTTCTGTAGCCAGTCTACATGTGTGCCACC TCTGACCTTTGGCGCTGGCACAAAGCTGGAACTGAAGCGG |
| 51 | RNA | anti-GD2 scFv 14G2a V227C | GCTGCAGTCTGGCCCTGAACTGGAAAAACCTGGCGCCTCCGTG ATGATCAGCTGTAAAGCCAGCGGCAGCAGCTTCACCGGCTACA ACATGAACTGGGTCCGACAGAACATCGGCAAGAGCCTGGAATG GATCGGCGCCATCGATCCTTACTACGGCGGCACCAGCTACAAC CAGAAGTTCAAGGGCAGAGCCACACTGACCGTGGACAAGAGCA GCAGCACAGCCTACATGCACCTGAAGTCCCTGACAAGCGAGGA CAGCGCCGTGTACTACTGTGTGTCCGGCATGGAATATTGGGGC CAGGGCACAAGCGTGACAGTTTCTTCTGGTGGCGGAGGATCTG GCGGAGGTGGAAGCGGCGGAGGCGGATCTGAAATCGTGATGA CACAGAGCCCCGCCACTCTGAGTGTGTCTCCAGGCGAAAGAGC TACCCTGAGCTGTAGAAGCAGCCAGAGCCTGGTGCACAGAAAC GGCAATACCTACCTGCACTGGTATCTGCAGAAGCCCGGCCAGT CTCCTAAGCTGCTGATCCACAAGGTGTCCAACAGATTCAGCGG CGTGCCCGATAGATTTTCTGGCTCTGGCAGCGGCACCGACTTC ACCCTGAAGATTAGCAGAGTGGAAGCCGAGGACCTGGGCGTG TACTTCTGTAGCCAGTCTACACACTGTCCACCTCTGACCTTTGG CGCTGGCACAAAGCTGGAACTGAAGCGG |
| 52 | DNA | EQ_CAR_FW, primer for sequencing and cloning of CAR constructs | TTCAAAGTTTTTTTCTTCCATTTCAGG |

(continued)

| SEQ ID NO | Type | Description | Sequence |
|---|---|---|---|
| 53 | DNA | SEQ_CAR_RV, primer for sequencing and cloning of CAR constructs | ACAAATTTTGTAATCCAGAGGTTGATT |
| 54 | DNA | AMP_CAR_KpnI_RV , for cloning of shorter CAR construct (GFP deletion) | TTATTAGGTACCGCGGGGAGGC |
| 55 | DNA | C105S_FW, for cloning of CB2 mutant C105S | CGCTACAGCGGCAAC |
| 56 | DNA | C105S_RV, for cloning of CB2 mutant C105S | GTTGCCGCTGTAGCG |
| 57 | DNA | 5' LTR (truncated) | GGGTCTCTCTGGTTAGACCAGATCTGAGCCTGGGAGCTCTCTG GCTAACTAGGGAACCCACTGCTTAAGCCTCAATAAAGCTTGCC TTGAGTGCTTCAAGTAGTGTGTGCCCGTCTGTTGTGTGACTCT GGTAACTAGAGATCCCTCAGACCCTTTTAGTCAGTGTGGAAAA TCTCTAGCA |
| 58 | DNA | 3' LTR (ΔU3) | TGGAAGGGCTAATTCACTCCCAACGAAGACAAGATCTGCTTTT TGCTTGTACTGGGTCTCTCTGGTTAGACCAGATCTGAGCCTGG GAGCTCTCTGGCTAACTAGGGAACCCACTGCTTAAGCCTCAAT AAAGCTTGCCTTGAGTGCTTCAAGTAGTGTGTGCCCGTCTGTT GTGTGACTCTGGTAACTAGAGATCCCTCAGACCCTTTTAGTCA GTGTGGAAAATCTCTAGCA |
| 59 | DNA | EF-1α promoter | GGCTCCGGTGCCCGTCAGTGGGCAGAGCGCACATCGCCCACA GTCCCCGAGAAGTTGGGGGGAGGGGTCGGCAATTGAACCGGT GCCTAGAGAAGGTGGCGCGGGGGTAAACTGGGAAAGTGATGTC GTGTACTGGCTCCGCCTTTTTCCCGAGGGTGGGGGAGAACCGT ATATAAGTGCAGTAGTCGCCGTGAACGTTCTTTTTTCGCAACGG GTTTGCCGCCAGAACACAGGTAAGTGCCGTGTGTGGTTCCCGC GGGCCTGGCCTCTTTACGGGTTATGGCCCTTGCGTGCCTTGAA TTACTTCCACCTGGCTGCAGTACGTGATTCTTGATCCCGAGCT |

(continued)

| SEQ ID NO | Type | Description | Sequence |
|---|---|---|---|
| | | | TCGGGTTGGAAGTGGGTGGGAGAGTTCGAGGCCTTGCGCTTA AGGAGCCCCTTCGCCTCGTGCTTGAGTTGAGGCCTGGCCTGG GCGCTGGGGCCGCCGCGTGCGAATCTGGTGGCACCTTCGCGC CTGTCTCGCTGCTTTCGATAAGTCTCTAGCCATTTAAAATTTTT GATGACCTGCTGCGACGCTTTTTTTCTGGCAAGATAGTCTTGT AAATGCGGGCCAAGATCTGCACACTGGTATTTCGGTTTTTGGG GCCGCGGGCGGCGACGGGGCCCGTGCGTCCCAGCGCACATGT TCGGCGAGGCGGGGCCTGCGAGCGCGGCCACCGAGAATCGGA CGGGGGTAGTCTCAAGCTGGCCGGCCTGCTCTGGTGCCTGGT CTCGCGCCGCCGTGTATCGCCCCGCCCTGGGCGGCAAGGCTG GCCCGGTCGGCACCAGTTGCGTGAGCGGAAAGATGGCCGCTT CCCGGCCCTGCTGCAGGGAGCTCAAAATGGAGGACGCGGCGC TCGGGAGAGCGGGCGGGTGAGTCACCCACACAAAGGAAAAGG GCCTTTCCGTCCTCAGCCGTCGCTTCATGTGACTCCACGGAGT ACCGGGCGCCGTCCAGGCACCTCGATTAGTTCTCGAGCTTTTG GAGTACGTCGTCTTTAGGTTGGGGGGGAGGGGTTTTATGCGAT GGAGTTTCCCCACACTGAGTGGGTGGAGACTGAAGTTAGGCCA GCTTGGCACTTGATGTAATTCTCCTTGGAATTTGCCCTTTTTGA GTTTGGATCTTGGTTCATTCTCAAGCCTCAGACAGTGGTTCAA AGTTTTTTTCTTCCATTTCAGGTGTCGTGA |
| 60 | DNA | CMV promoter | GTGATGCGGTTTTGGCAGTACATCAATGGGCGTGGATAGCGG TTTGACTCACGGGGATTTCCAAGTCTCCACCCCATTGACGTCA ATGGGAGTTTGTTTTGGCACCAAAATCAACGGGACTTTCCAAA ATGTCGTAACAACTCCGCCCCATTGACGCAAATGGGCGGTAGG CGTGTACGGTGGGAGGTCTATATAAGCAGAGCT |

36

(continued)

| SEQ ID NO | Type | Description | Sequence |
|-----------|------|-------------|----------|
| 61 | DNA | WPRE | AATCAACCTCTGGATTACAAAATTTGTGAAAGATTGACTGGTAT TCTTAACTATGTTGCTCCTTTTACGCTATGTGGATACGCTGCTT TAATGCCTTTGTATCATGCTATTGCTTCCCGTATGGCTTTCATT TTCTCCTCCTTGTATAAATCCTGGTTGCTGTCTCTTTATGAGGA GTTGTGGCCCGTTGTCAGGCAACGTGGCGTGGTGTGCACTGT GTTTGCTGACGCAACCCCCACTGGTTGGGGCATTGCCACCACC TGTCAGCTCCTTTCCGGGACTTTCGCTTTCCCCCTCCCTATTGC CACGGCGGAACTCATCGCCGCCTGCCTTGCCCGCTGCTGGACA GGGGCTCGGCTGTTGGGCACTGACAATTCCGTGGTGTTGTCG GGGAAGCTGACGTCCTTTCCATGGCTGCTCGCCTGTGTTGCCA CCTGGATTCTGCGCGGGACGTCCTTCTGCTACGTCCCTTCGGC CCTCAATCCAGCGGACCTTCCTTCCCGCGGCCTGCTGCCGGCT CTGCGGCCTCTTCCGCGTCTTCGCCTTCGCCCTCAGACGAGTC GGATCTCCCTTTGGGCCGCCTCCCCGC |

The sequences in the Table of Sequences are presented according to WIPO Standard ST.26, which lists RNA sequences with the nucleotide T instead of U. However, these RNA sequences should be interpreted and utilized as containing the U nucleotide at T positions.

## References

1. Abreu TR, Fonseca NA, Gonçalves N, Moreira JN. Current challenges and emerging opportunities of CAR-T cell therapies. Journal of Controlled Release. 2020;319:246-261.

2. Frey N, Porter D. Cytokine Release Syndrome with Chimeric Antigen Receptor T Cell Therapy. Biology of Blood and Marrow Transplantation. 2019;25(4):e123-e127.

3. Gust J, Hay KA, Hanafi LA, et al. Endothelial activation and blood-brain barrier disruption in neurotoxicity after adoptive immunotherapy with CD19 CAR-T cells. Cancer Discov. 2017;7(12):1404-1419.

4. Rodriguez-Garcia A, Palazon A, Noguera-Ortega E, Powell DJ, Guedan S. CAR-T Cells Hit the Tumor Microenvironment: Strategies to Overcome Tumor Escape. Front Immunol. 2020;11(1109):1-17.

5. Brassart-Pasco S, Brézillon S, Brassart B, et al. Tumor Microenvironment: Extracellular Matrix Alterations Influence Tumor Progression. Front Oncol. 2020;10(397): 1-13.

6. Xu S, Sankar S, Neamati N. Protein disulfide isomerase: a promising target for cancer therapy. Drug Discov Today. 2014;19(3):222-240.

7. Arnér ESJ, Holmgren A. The thioredoxin system in cancer. Semin Cancer Biol. 2006;16(6):420-426.

8. Goerdeler F, Reuber EE, Lühle J, et al. Thiol-Mediated Uptake of a Cysteine-Containing Nanobody for Anticancer Drug Delivery. ACS Cent Sci. 2023;9(6):1111-1118.

9. Popielarski M, Ponamarczuk H, Stasiak M, Wataka C, Świątkowska M. Modifications of disulfide bonds in breast cancer cell migration and invasiveness. *Am J Cancer Res.* 2019;9(8):1554-1582.

10. Ceccarelli J, Delfino L, Zappia E, et al. The redox state of the lung cancer microenvironment depends on the levels of thioredoxin expressed by tumor cells and affects tumor progression and response to prooxidants. Int J Cancer. 2008;123(8):1770-1778.

11. Tufo G, Jones AWE, Wang Z, et al. The protein disulfide isomerases PDIA4 and PDIA6 mediate resistance to cisplatin-induced cell death in lung adenocarcinoma. Cell Death Differ. 2014;21(5):685-695.

12. Kuo TF, Chen TY, Jiang ST, et al. Protein disulfide isomerase a4 acts as a novel regulator of cancer growth through the procaspase pathway. Oncogene. 2017;36(39):5484-5496.

13. Robinson RM, Reyes L, Duncan RM, et al. Inhibitors of the protein disulfide isomerase family for the treatment of multiple myeloma. Leukemia. 2019;33(4):1011-1022.

14. Chen, W., Yuan, Y., & Jiang, X. (2020). Antibody and antibody fragments for cancer immunotherapy. Journal of Controlled Release, 328, 395-406.

15. van de Donk, N. W., & Zweegman, S. (2023). T-cell-engaging bispecific antibodies in cancer. The Lancet, 402(10396), 142-158.

16. Lu, R. M., Hwang, Y. C., Liu, I. J., Lee, C. C., Tsai, H. Z., Li, H. J., & Wu, H. C. (2020). Development of therapeutic antibodies for the treatment of diseases. Journal of biomedical science, 27, 1-30.

17. Delgado, M., & Garcia-Sanz, J. A. (2023). Therapeutic Monoclonal Antibodies against Cancer: Present and Future. Cells, 12(24), 2837.

18. Torka, P., Barth, M., Ferdman, R., & Hernandez-Ilizaliturri, F. J. (2019). Mechanisms of resistance to monoclonal antibodies (mAbs) in lymphoid malignancies. Current hematologic malignancy reports, 14, 426-438.

19. Van Cutsem, E., Paccard, C., Chiron, M., & Tabernero, J. (2020). Impact of prior bevacizumab treatment on VEGF-A and PIGF levels and outcome following second-line aflibercept treatment: biomarker post hoc analysis of the VELOUR trial. Clinical Cancer Research, 26(3), 717-725.

**Claims**

1. An antigen-binding domain comprising a heavy chain variable region (VH), and optionally a light chain variable region (VL); wherein the antigen binding domain is capable of binding to an antigen,

   **characterised in that** the VH, and optionally the VL, comprise three complementarity-determining regions (CDRs), wherein at least one CDR comprises at least one amino acid residue other than cysteine, which has a solvent-accessible surface area from 0.00 to 0.75 and is located at a distance to the antigen from 3 to 14 Å, and wherein said at least one amino acid residue is substituted to a thiol-group containing amino acid.

2. The antigen-binding domain of claim 1, wherein the solvent-accessible surface area is from 0.00 to 0.70, more preferably area is from 0.00 to 0.65, even more preferably is from 0.00 to 0.60, even more preferably is from 0.00 to 0.55, and most preferably is from 0.00 to 0.50.

3. The antigen-binding domain of any one of the preceding claims, wherein the residue for cysteine substitution is located at the distance to the antigen of 3-13 Å, more preferably of 3.5-12 Å, even more preferably of 4-11 Å, even more preferably of 4-10 Å, even more preferably of 4-9 Å, and most preferably of 4-8 Å.

4. The antigen-binding domain of any one of the preceding claims, wherein the solvent accessibility and the distance to the antigen are defined based on a structural model of the antigen and the antigen binding domain, wherein the structural model is an X-ray structure, a cryo-EM-based model, or an AI-generated model, preferably an X-ray structure.

5. The antigen-binding domain of any one of the preceding claims, wherein the antigen-binding domain binds the antigen with at least 1 mM $K_d$, wherein the $K_d$ is determinable by surface plasmon resonance (SPR), enzyme-linked immunosorbent assay (ELISA), Isothermal Titration Calorimetry (ITC), or MicroScale Thermophoresis (MST).

6. The antigen-binding domain of any one of the preceding claims, wherein the thiol-group containing amino acid is selected from cysteine, cysteamine, 3-mercapto propionic acid, cysteine sulfinic acid, S-sulfocysteine, N-acetylcysteine (NAC), N, N'-Diacetyl-L-cystine, N, N'-Diacetyl-L-cystine Dimethylester (DACDM), S-Propylcysteine, S-Allylcysteine, S-propargyl-cysteine (SPRC), and S-Phenylcysteine, preferably wherein the thiol-group containing amino acid is cysteine.

7. The antigen-binding domain of any one of the preceding claims, wherein the antigen binding domain is part of an antibody, a nanobody, a single chain variable fragment, or a Bi-specific T-cell engager; or
wherein the antigen binding domain forms an antibody together with constant domains, a hinge region and a Fc region, preferably wherein the antibody is an IgG antibody, more preferably wherein the antibody is an IgG1 or IgG4 antibody, even more preferably wherein the antibody is an IgG1 antibody.

8. The antigen-binding domain of any one of the preceding claims, wherein the antigen-binding domain binds to a target cell in an antigen-dependent manner, preferably wherein the antigen-binding domain additionally binds to the target in an antigen-independent manner, preferably wherein the antigen-independent binding comprises formation of at least one bond between at least one thiol group containing amino acid residue within at least one CDR of the antigen-binding domain and at least one cysteine residue present in at least one surface protein of a cell.

9. The antigen-binding domain according to any of claims 1-8 for use in a method of treating a cancer, preferably wherein the cancer comprises a subset of cancer cells that is **characterized in** a reduced expression of the antigen, in particular wherein the cancer cells have undergone an antigen escape or wherein the cancer has a risk of undergoing antigen-escape.

10. The antigen-binding domain for use in the method of treatment according to claim 9, wherein the cancer comprises a subset of cancer cells that do not express the antigen targeted by the antigen-binding domain, preferably wherein said antigen-negative subset is at least 0.5% of the total cancer cell population as determinable by FACS.

11. The antigen-binding domain for use in the method of treatment according to claims 9 or 10, wherein:
the cancer cell targeted in said method of treatment has at least a 2-fold increase in the expression level of at least one enzyme that catalyzes oxidation of the thiol-group containing amino acid residues, compared to the expression level of said enzyme in a non-cancerous cell of the same cell type.

12. The antigen-binding domain for use in the method of treatment according to any one of the claims 9-11, wherein the cancer cell targeted in said method of treatment expresses CD19 or GD2 on its surface.

13. The antigen-binding domain for use in the method of treatment according to claim 33, wherein the hematological cancer is a lymphoma or a leukemia.

14. The antigen-binding domain according to any of claims 1-8 for use in an immunomodulating therapy.

15. A method of generating an antigen-binding domain

   comprising a heavy chain variable region (VH) and, optionally, also a light chain variable region (VL); and wherein the method comprises the steps of:

   a) selecting at least one amino acid residue suitable for cysteine substitution, wherein said at least one amino acid residue is comprised within a CDR of VH or VL, has a solvent-accessible surface area from 0.00 to 0.75 and is located at a distance to the antigen from 3 to 14 A, as defined based on a structural model of an antigen and antigen binding domain
   b) mutating selected residue to a thiol-group containing amino acid.

## Fig. 1A

CB2-CAR-T Cell       B Cell Lymphoma

## Fig. 1B

## Fig. 1C

**Fig. 1D**

**Fig. 2A**

**Fig. 2B**

## Fig. 2C

## Fig. 2D

## Fig. 3A

## Fig. 3B

## Fig. 3C

## Fig. 4A

## Fig. 4B

## Fig. 4C

**Fig. 5A**

**Fig. 5B**

**Fig. 5C**

## Fig. 5D

## Fig. 6A

Solvent-accessible surface area (SASA)

# Fig. 6B

CD19
FMC63-VH
FMC63-VL

Distance to antigen

## Fig. 7A

tumor cell injection on day -6

**Fig. 7B**

**Fig. 7C**

## Fig. 8 A-D

**A**

| 5' LTR | EF-1α | SP | CB2 | IgG1-Fc | CD28-TM | CD28 | CD3ζ | WPRE | CMV | GFP | 3' LTR |

**B**

| 5' LTR | CMV | GFP | 3' LTR |

**C**

| 5' LTR | EF-1α | SP | CB2 | IgG1-Fc | CD28-TM | CD28 | CD3ζ | WPRE | 3' LTR |

| 5' LTR | EF-1α | SP | C105S CB2 | IgG1-Fc | CD28-TM | CD28 | CD3ζ | WPRE | 3' LTR |

| 5' LTR | EF-1α | SP | LaG-16 | IgG1-Fc | CD28-TM | CD28 | CD3ζ | WPRE | 3' LTR |

| 5' LTR | EF-1α | SP | S108C LaG-16 | IgG1-Fc | CD28-TM | CD28 | CD3ζ | WPRE | 3' LTR |

**D**

| 5' LTR | EF-1α | SP | CB2 | CD8-h | CD8-TM | 4-1BB | CD3ζ | WPRE | 3' LTR |

| 5' LTR | EF-1α | SP | FMC63 | CD8-h | CD8-TM | 4-1BB | CD3ζ | WPRE | 3' LTR |

## Fig. 9

## Fig. 10 A

## Fig. 10 B

## Fig. 11

# Fig. 12

# Fig. 13 A-B

Europäisches Patentamt

European Patent Office

Office européen des brevets

# EUROPEAN SEARCH REPORT

**Application Number**

EP 24 19 9456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/124316 A2 (IRM LLC [US]; GEIERSTANGER BERNHARD HUBERT [US] ET AL.) 14 August 2014 (2014-08-14) | 1-3, 5-11,14, 15 | INV. A61K40/11 A61K40/31 A61K40/42 C07K16/28 C07K16/30 |
| Y | * pages 114, 121; figure 1; examples 1-3; tables 6, 20 * | 4,12 | |
| | ----- | | |
| X | WO 2011/156328 A1 (GENENTECH INC [US]; BHAKTA SUNIL [US]; JUNUTULA JAGATH R [US]) 15 December 2011 (2011-12-15) | 1-11, 13-15 | |
| Y | * paragraph [0146] - paragraph [0153]; tables 1,2 * | 12 | |
| | ----- | | |
| X | WO 2022/198335 A1 (ZYMEWORKS INC [CA]) 29 September 2022 (2022-09-29) | 1-3, 5-11,14, 15 | |
| Y | * paragraphs [0007], [0180]; example 1; table 8.1 * | 4,12 | |
| | ----- | | |
| X | STADLMAYR GERHARD ET AL: "Efficient spontaneous site-selective cysteine-mediated toxin attachment within a structural loop of antibodies", BIOCHIMICA ET BIOPHYSICA ACTA, ELSEVIER, AMSTERDAM, NL, vol. 1866, no. 7, 22 April 2022 (2022-04-22), XP087043630, ISSN: 0304-4165, DOI: 10.1016/J.BBAGEN.2022.130155 [retrieved on 2022-04-22] | 1-3,6-8 | TECHNICAL FIELDS SEARCHED (IPC) A61K C07K |
| Y | * tables 1,2 * | 4,9-15 | |
| | ----- | | |
| X | WO 2019/068756 A1 (MERCK PATENT GMBH [DE]; UNIV WIEN BODENKULTUR [AT]) 11 April 2019 (2019-04-11) | 1-3, 6-11,14, 15 | |
| Y | * page 76 - page 78; example 10; table 24 * | 4,12 | |
| | ----- | | |
| | -/-- | | |

The present search report has been drawn up for all claims

| Place of search | Date of completion of the search | Examiner |
|---|---|---|
| The Hague | 7 March 2025 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 9456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/157595 A1 (MEDIMMUNE LLC [US]) 15 October 2015 (2015-10-15) * table 6 * ----- | 1-15 | |
| X | WO 2009/052249 A1 (GENENTECH INC [US]; MAO WEIGUANG [US] ET AL.) 23 April 2009 (2009-04-23) | 1-3, 6-11,14, 15 | |
| Y | * pages 5, 47 - page 48 * ----- | 4,12 | |
| X | WANG YUEDI ET AL: "Chimeric antigen receptor clustering via cysteines enhances T-cell efficacy against tumor", CANCER IMMUNOLOGY IMMUNOTHERAPY, vol. 71, no. 11, 1 November 2022 (2022-11-01), pages 2801-2814, XP093244660, Berlin/Heidelberg ISSN: 0340-7004, DOI: 10.1007/s00262-022-03195-4 Retrieved from the Internet: URL:https://link.springer.com/content/pdf/10.1007/s00262-022-03195-4.pdf> * page 2805 - page 2807; figures 1A, 2J * ----- | 1-3,6-8 | |
| Y | GOERDELER FELIX ET AL: "Thiol-Mediated Uptake of a Cysteine-Containing Nanobody for Anticancer Drug Delivery", ACS CENTRAL SCIENCE, vol. 9, no. 6, 28 June 2023 (2023-06-28), pages 1111-1118, XP093244483, ISSN: 2374-7943, DOI: 10.1021/acscentsci.3c00177 Retrieved from the Internet: URL:https://pubs.acs.org/doi/pdf/10.1021/acscentsci.3c00177> * page 1117; figure S2A * ----- | 1-15 | TECHNICAL FIELDS SEARCHED (IPC) |

The present search report has been drawn up for all claims

| Place of search | Date of completion of the search | Examiner |
|---|---|---|
| The Hague | 7 March 2025 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 9456

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2025

| Patent document cited in search report | Publication date | Patent family member(s) | Publication date |
|---|---|---|---|
| WO 2014124316 A2 | 14-08-2014 | AU 2014214751 A1 | 20-08-2015 |
| | | AU 2017219059 A1 | 14-09-2017 |
| | | BR 112015018899 A2 | 06-08-2015 |
| | | CA 2900755 A1 | 14-08-2014 |
| | | CN 105143271 A | 09-12-2015 |
| | | CN 115925957 A | 07-04-2023 |
| | | DK 2953976 T3 | 21-06-2021 |
| | | EA 201591465 A1 | 30-12-2015 |
| | | EP 2953976 A2 | 16-12-2015 |
| | | EP 3929217 A2 | 29-12-2021 |
| | | ES 2874493 T3 | 05-11-2021 |
| | | HK 1213580 A1 | 08-07-2016 |
| | | HU E054443 T2 | 28-09-2021 |
| | | JP 6609477 B2 | 20-11-2019 |
| | | JP 2016511637 A | 21-04-2016 |
| | | KR 20150115000 A | 13-10-2015 |
| | | KR 20210125593 A | 18-10-2021 |
| | | KR 20220133313 A | 04-10-2022 |
| | | PL 2953976 T3 | 02-11-2021 |
| | | PT 2953976 T | 23-06-2021 |
| | | SG 10201706468R A | 28-09-2017 |
| | | SG 11201506025R A | 28-08-2015 |
| | | SI 2953976 T1 | 31-08-2021 |
| | | US 2016067351 A1 | 10-03-2016 |
| | | US 2020338207 A1 | 29-10-2020 |
| | | US 2023270876 A1 | 31-08-2023 |
| | | WO 2014124316 A2 | 14-08-2014 |
| WO 2011156328 A1 | 15-12-2011 | AU 2011265054 A1 | 15-11-2012 |
| | | AU 2016256788 A1 | 01-12-2016 |
| | | BR 112012030311 A2 | 24-01-2017 |
| | | CA 2799540 A1 | 15-12-2011 |
| | | CA 3220104 A1 | 15-12-2011 |
| | | CN 103068406 A | 24-04-2013 |
| | | CN 107335062 A | 10-11-2017 |
| | | CN 114246952 A | 29-03-2022 |
| | | CN 119552249 A | 04-03-2025 |
| | | EP 2579897 A1 | 17-04-2013 |
| | | JP 2013534520 A | 05-09-2013 |
| | | JP 2018027932 A | 22-02-2018 |
| | | KR 20130087382 A | 06-08-2013 |
| | | MX 336540 B | 22-01-2016 |
| | | RU 2012156248 A | 20-07-2014 |
| | | RU 2017124859 A | 31-01-2019 |
| | | SG 185428 A1 | 28-12-2012 |
| | | SG 10201600791T A | 30-03-2016 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 24 19 9456

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2025

| Patent document cited in search report | Publication date | Patent family member(s) | Publication date |
|---|---|---|---|
| | | US 2011301334 A1 | 08-12-2011 |
| | | US 2014288280 A1 | 25-09-2014 |
| | | US 2017260253 A1 | 14-09-2017 |
| | | US 2020339664 A1 | 29-10-2020 |
| | | US 2024218050 A1 | 04-07-2024 |
| | | WO 2011156328 A1 | 15-12-2011 |
| WO 2022198335 A1 | 29-09-2022 | AU 2022243931 A1 | 09-11-2023 |
| | | BR 112023019698 A2 | 27-02-2024 |
| | | CA 3178093 A1 | 26-09-2022 |
| | | CN 117242091 A | 15-12-2023 |
| | | EP 4314043 A1 | 07-02-2024 |
| | | IL 307231 A | 01-11-2023 |
| | | JP 2024510526 A | 07-03-2024 |
| | | KR 20230171944 A | 21-12-2023 |
| | | WO 2022198335 A1 | 29-09-2022 |
| WO 2019068756 A1 | 11-04-2019 | AU 2018346264 A1 | 23-04-2020 |
| | | CA 3076867 A1 | 11-04-2019 |
| | | CN 111683963 A | 18-09-2020 |
| | | EP 3692060 A1 | 12-08-2020 |
| | | IL 273761 A | 31-05-2020 |
| | | JP 7543134 B2 | 02-09-2024 |
| | | JP 2021500873 A | 14-01-2021 |
| | | US 2020277399 A1 | 03-09-2020 |
| | | WO 2019068756 A1 | 11-04-2019 |
| | | WO 2019068758 A1 | 11-04-2019 |
| WO 2015157595 A1 | 15-10-2015 | AU 2015243380 A1 | 10-11-2016 |
| | | BR 112016023436 A2 | 17-10-2017 |
| | | CA 2944784 A1 | 15-10-2015 |
| | | CN 106459205 A | 22-02-2017 |
| | | CY 1122155 T1 | 25-11-2020 |
| | | DK 3129406 T3 | 01-04-2019 |
| | | EP 3129406 A1 | 15-02-2017 |
| | | ES 2719584 T3 | 11-07-2019 |
| | | HR P20190486 T1 | 31-05-2019 |
| | | HU E041976 T2 | 28-06-2019 |
| | | JP 6685924 B2 | 22-04-2020 |
| | | JP 2017512486 A | 25-05-2017 |
| | | LT 3129406 T | 10-04-2019 |
| | | PL 3129406 T3 | 31-07-2019 |
| | | PT 3129406 T | 24-04-2019 |
| | | SI 3129406 T1 | 30-04-2019 |
| | | SM T201900160 T1 | 10-05-2019 |
| | | TR 201903526 T4 | 21-03-2019 |

EPO FORM P0459

For more details about this annex : see Official Journal of the European Patent Office, No. 12/82

page 2 of 3

## ANNEX TO THE EUROPEAN SEARCH REPORT
### ON EUROPEAN PATENT APPLICATION NO.

EP 24 19 9456

This annex lists the patent family members relating to the patent documents cited in  the above-mentioned European search report.
The members are as contained in the European Patent Office EDP file on
The European Patent Office is in no way liable for these particulars which are merely given for the purpose of information.

07-03-2025

| Patent document cited in search report | Publication date | Patent family member(s) | Publication date |
|---|---|---|---|
| | | US 2018169255 A1 | 21-06-2018 |
| | | US 2021069341 A1 | 11-03-2021 |
| | | WO 2015157595 A1 | 15-10-2015 |
| WO 2009052249 A1 | 23-04-2009 | AR 068941 A1 | 16-12-2009 |
| | | AU 2008312457 A1 | 23-04-2009 |
| | | AU 2014203779 A1 | 31-07-2014 |
| | | BR PI0818780 A2 | 22-04-2015 |
| | | CA 2698541 A1 | 23-04-2009 |
| | | CL 2008003074 A1 | 28-12-2009 |
| | | CN 101835803 A | 15-09-2010 |
| | | CO 6390071 A2 | 29-02-2012 |
| | | EP 2209808 A1 | 28-07-2010 |
| | | ES 2450755 T3 | 25-03-2014 |
| | | HK 1143379 A1 | 31-12-2010 |
| | | IL 204159 A | 29-02-2016 |
| | | JP 5606916 B2 | 15-10-2014 |
| | | JP 2011504460 A | 10-02-2011 |
| | | JP 2013173776 A | 05-09-2013 |
| | | KR 20100090267 A | 13-08-2010 |
| | | MY 188455 A | 09-12-2021 |
| | | NZ 584514 A | 27-07-2012 |
| | | PE 20091112 A1 | 25-07-2009 |
| | | PE 20140833 A1 | 14-07-2014 |
| | | RU 2010119937 A | 27-11-2011 |
| | | TW 200927172 A | 01-07-2009 |
| | | US 2009117100 A1 | 07-05-2009 |
| | | US 2015158952 A1 | 11-06-2015 |
| | | US 2017166635 A1 | 15-06-2017 |
| | | WO 2009052249 A1 | 23-04-2009 |
| | | ZA 201001383 B | 28-04-2011 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ABREU TR** ; **FONSECA NA** ; **GONÇALVES N** ; **MOREIRA JN**. Current challenges and emerging opportunities of CAR-T cell therapies. *Journal of Controlled Release*, 2020, vol. 319, 246-261
- **FREY N** ; **PORTER D**. Cytokine Release Syndrome with Chimeric Antigen Receptor T Cell Therapy. *Biology of Blood and Marrow Transplantation*, 2019, vol. 25 (4), e123-e127
- **GUST J** ; **HAY KA** ; **HANAFI LA et al.** Endothelial activation and blood-brain barrier disruption in neurotoxicity after adoptive immunotherapy with CD19 CAR-T cells. *Cancer Discov.*, 2017, vol. 7 (12), 1404-1419
- **RODRIGUEZ-GARCIA A** ; **PALAZON A** ; **NOGUERA-ORTEGA E** ; **POWELL DJ** ; **GUEDAN S**. CAR-T Cells Hit the Tumor Microenvironment: Strategies to Overcome Tumor Escape. *Front Immunol.*, 2020, vol. 11 (1109), 1-17
- **BRASSART-PASCO S** ; **BRÉZILLON S** ; **BRASSART B et al.** Tumor Microenvironment: Extracellular Matrix Alterations Influence Tumor Progression. *Front Oncol.*, 2020, vol. 10 (397), 1-13
- **XU S** ; **SANKAR S** ; **NEAMATI N**. Protein disulfide isomerase: a promising target for cancer therapy. *Drug Discov Today.*, 2014, vol. 19 (3), 222-240
- **ARNÉR ESJ** ; **HOLMGREN A**. The thioredoxin system in cancer. *Semin Cancer Biol.*, 2006, vol. 16 (6), 420-426
- **GOERDELER F** ; **REUBER EE** ; **LÜHLE J et al.** Thiol-Mediated Uptake of a Cysteine-Containing Nanobody for Anticancer Drug Delivery. *ACS Cent Sci.*, 2023, vol. 9 (6), 1111-1118
- **CECCARELLI J** ; **DELFINO L** ; **ZAPPIA E et al.** The redox state of the lung cancer microenvironment depends on the levels of thioredoxin expressed by tumor cells and affects tumor progression and response to prooxidants. *Int J Cancer.*, 2008, vol. 123 (8), 1770-1778
- **TUFO G** ; **JONES AWE** ; **WANG Z et al.** The protein disulfide isomerases PDIA4 and PDIA6 mediate resistance to cisplatin-induced cell death in lung adenocarcinoma. *Cell Death Differ.*, 2014, vol. 21 (5), 685-695
- **KUO TF** ; **CHEN TY** ; **JIANG ST et al.** Protein disulfide isomerase a4 acts as a novel regulator of cancer growth through the procaspase pathway. *Oncogene*, 2017, vol. 36 (39), 5484-5496
- **ROBINSON RM** ; **REYES L** ; **DUNCAN RM et al.** Inhibitors of the protein disulfide isomerase family for the treatment of multiple myeloma. *Leukemia*, 2019, vol. 33 (4), 1011-1022
- **CHEN, W.** ; **YUAN, Y.** ; **JIANG, X.** Antibody and antibody fragments for cancer immunotherapy. *Journal of Controlled Release*, 2020, vol. 328, 395-406
- **VAN DE DONK, N. W.** ; **ZWEEGMAN, S.** T-cell-engaging bispecific antibodies in cancer. *The Lancet*, 2023, vol. 402 (10396), 142-158
- **LU, R. M.** ; **HWANG, Y. C.** ; **LIU, I. J.** ; **LEE, C. C.** ; **TSAI, H. Z.** ; **LI, H. J.** ; **WU, H. C.** Development of therapeutic antibodies for the treatment of diseases. *Journal of biomedical science*, 2020, vol. 27, 1-30
- **DELGADO, M.** ; **GARCIA-SANZ, J. A.** Therapeutic Monoclonal Antibodies against Cancer: Present and Future. *Cells*, 2023, vol. 12 (24), 2837
- **TORKA, P.** ; **BARTH, M.** ; **FERDMAN, R.** ; **HERNANDEZ-ILIZALITURRI, F. J.** Mechanisms of resistance to monoclonal antibodies (mAbs) in lymphoid malignancies. *Current hematologic malignancy reports*, 2019, vol. 14, 426-438
- **VAN CUTSEM, E.** ; **PACCARD, C.** ; **CHIRON, M.** ; **TABERNERO, J.** Impact of prior bevacizumab treatment on VEGF-A and PIGF levels and outcome following second-line aflibercept treatment: biomarker post hoc analysis of the VELOUR trial. *Clinical Cancer Research*, 2020, vol. 26 (3), 717-725